(19) Europäisches Patentamt
European Patent Office
Office européen des brevets

(11) Veröffentlichungsnummer: **0 381 133 B1**

(12) # EUROPÄISCHE PATENTSCHRIFT

(45) Veröffentlichungstag der Patentschrift: **02.03.94**

(51) Int. Cl.$^5$: **C09B 62/507**, D06P 1/384, C07C 317/28, C09B 29/01

(21) Anmeldenummer: **90101781.4**

(22) Anmeldetag: **30.01.90**

(54) **Wasserlösliche Azoverbindungen, Verfahren zu ihrer Herstellung und ihre Verwendung als Farbstoffe.**

(30) Priorität: **03.02.89 DE 3903224**

(43) Veröffentlichungstag der Anmeldung:
**08.08.90 Patentblatt 90/32**

(45) Bekanntmachung des Hinweises auf die Patenterteilung:
**02.03.94 Patentblatt 94/09**

(84) Benannte Vertragsstaaten:
**CH DE GB LI**

(56) Entgegenhaltungen:
**EP-A- 0 238 894**
**DE-A- 2 126 143**
**DE-A- 3 536 688**
**DE-A- 3 624 136**

(73) Patentinhaber: **HOECHST AKTIENGESELLSCHAFT**

**D-65926 Frankfurt(DE)**

(72) Erfinder: **Springer, Hartmut, Dr.**
**Am Erdbeerstein 27**
**D-6240 Königstein/Taunus(DE)**
Erfinder: **Reiher, Uwe, Dr.**
**Koblenzer Strasse 52**
**D-6000 Frankfurt am Main(DE)**

**EP 0 381 133 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung liegt auf dem Gebiet der faserreaktiven Farbstoffe.

Es wurden neue wasserlösliche Azoverbindungen entsprechend der allgemeinen Formel (1)

D — N = N — K (1)

gefunden, die wertvolle faserreaktive Farbstoffeigenschaften besitzen.

In dieser Formel (1) bedeuten:

D ist ein Rest der allgemeinen Formel (2)

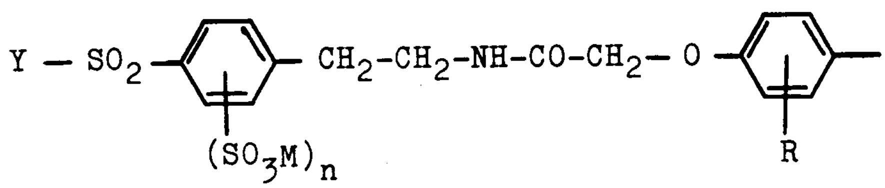

(2)

in welcher

Y die Vinylgruppe bedeutet oder eine Gruppe der allgemeinen Formel (3)

$-CH_2-CH_2-X$ (3)

in welcher

X ein Substituent ist, der durch ein Alkali unter Bildung der Vinylgruppe eliminierbar ist,

R ein Wasserstoffatom, eine Nitrogruppe, eine Alkylgruppe von 1 bis 4 C-Atomen, wie Ethyl- und insbesondere Methylgruppe, eine Alkoxygruppe von 1 bis 4 C-Atomen, wie Ethoxy- und insbesondere Methoxygruppe, eine Carboxygruppe, eine Hydroxygruppe oder ein Halogenatom, wie Chlor- und Bromatom, bevorzugt jedoch ein Wasserstoffatom ist,

n für die Zahl Null oder 1, bevorzugt Null, steht (wobei im Fall n = Null die Gruppe ein Wasserstoffatom bedeutet) und

M ein Wasserstoffatom oder ein salzbildendes Metallatom, wie insbesondere ein Alkalimetallatom, wie beispielsweise Natrium, Kalium oder Lithium, ist;

K ist ein Rest einer einfach ankuppelbaren wasserlöslichen Kupplungskomponente, die noch eine Azogruppe enthalten kann, oder der Rest einer doppelankuppelbaren wasserlöslichen Kupplungskomponente, jeweils aus der Reihe der Aminobenzole, der Phenole, insbesondere deren Sulfonsäuren und Carbonsäuren, der Naphthole, insbesondere deren Sulfonsäuren und Carbonsäuren, der Aminonaphthole, insbesondere deren Sulfonsäuren, der Acylamino-naphthole, insbesondere deren Sulfonsäuren, mit dem Acylrest einer Alkan- oder Alkencarbonsäure mit jeweils 1 bis 4 bzw. 2 bis 4 C-Atomen im Alkyl- bzw. Alkenylrest oder einer aromatischen Carbonsäure, wie der Benzoesäure, oder einer aromatischen Sulfonsäure, wie der Benzol- oder Toluolsulfonsäure, oder einer N-substituierten Carbaminsäure, wie der N-Phenylureido-Rest, oder aus der Reihe der Dihydroxynaphthalinsulfonsäuren, der Phenylazo-und Naphthylazo-aminonaphtholsulfonsäuren, der 5-Pyrazolone und 5-Aminopyrazole, der Acetoacetylarylide, der 2-Hydroxy-6-pyridone und der Hydroxychinoline, wobei K neben den in Farbstoffen üblichen Substituenten auch eine oder mehrere faserreaktive Gruppen enthalten kann, wie bspw. eine Gruppe $-SO_2-Y$ mit Y der obigen Bedeutung oder eine 4-Fluor- oder 4-Chlor-6-amino-s-triazin-2-ylamino-Gruppe,deren 6-ständige Aminogruppe durch Alkyl von 1 bis 4 C-Atomen und/oder Phenyl mono- oder disubstituiert sein kann, wobei der Phenylrest durch Substituenten aus der Gruppe Sulfo, Carboxy, Methoxy, Ethoxy, Methyl, Chlor, Brom und $-SO_2-Y$ mit Y der obigen Bedeutung substituiert sein kann.

Alkalisch eliminierbare Substituenten X sind beispielsweise Halogenatome, wie das Bromatom und Chloratom, Estergruppen organischer Carbon- und Sulfonsäuren, wie ein Alkanoyloxyrest von 2 bis 5 C-Atomen, beispielsweise die Acetyloxygruppe, oder ein Sulfobenzoyloxy-, Benzoyloxy-, Phenylsulfonyloxy- oder Toluylsulfonyloxy-Rest, desweiteren beispielsweise die sauren Estergruppen der Phosphorsäure, der Schwefelsäure und der Thioschwefelsäure (Phosphato- bzw. Sulfato- bzw. Thiosulfatogruppen), ebenso Dialkylaminogruppen mit Alkylgruppen von jeweils 1 bis 4 C-Atomen, wie die Dimethylamino- und Diethylaminogruppe.

Bevorzugt ist Y die Vinylgruppe und insbesondere die β-Sulfatoethyl-Gruppe.

Sulfogruppen sind Gruppen entsprechend der allgemeinen Formel $-SO_3M$ , Carboxygruppen sind Gruppen entsprechend der allgemeinen Formel -COOM , Sulfatogruppen sind Gruppen entsprechend der allgemeinen Formel $-OSO_3M$ , Phosphonogruppen sind Gruppen der allgemeinen Formel $-PO_3M_2$ , Thiosulfatogruppen sind Gruppen entsprechend der allgemeinen Formel $-S-SO_3M$ und Phosphatogruppen sind Gruppen entsprechend der allgemeinen Formel $-OPO_3M_2$ , wobei M die obengenannte Bedeutung hat.

Von den erfindungsgemäßen Verbindungen der allgemeinen Formel (1) können beispielsweise solche Verbindungen hervorgehoben werden, in welchen K einen Rest der nachstehenden Formel (4a), (4b), (4c), (4d), (4e), (4f), (4g), (4h), (4i), (4k), (4m), (4n), (4p), (4q), (4r), (4s), (4t) (4v) und (4w) bedeutet:

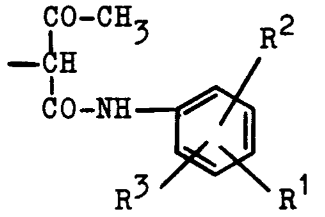

(4a)

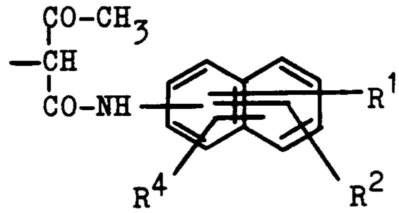

(4b)

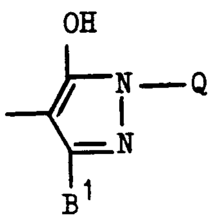

(4c)

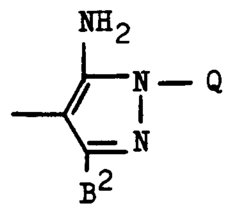

(4d)

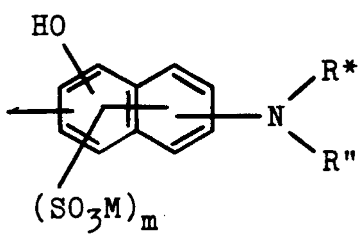

(4e)

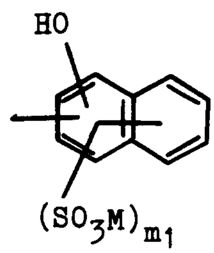

(4f)

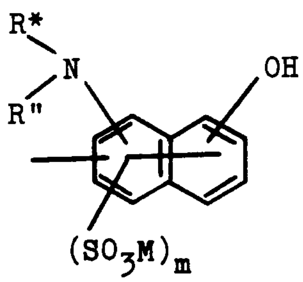

(4g)

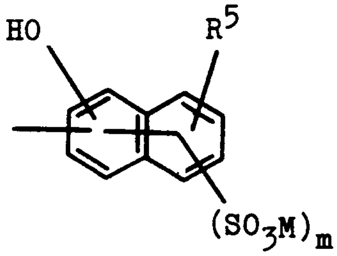

(4h)

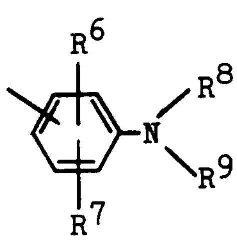

(4i)

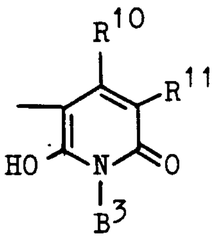
(4k)

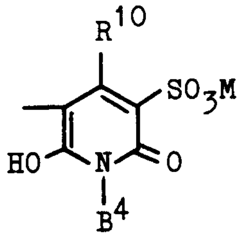
(4m)

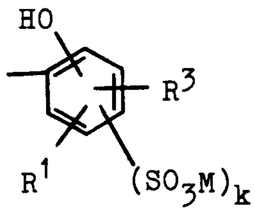
(4n)

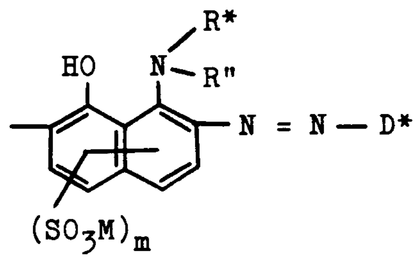
(4p)

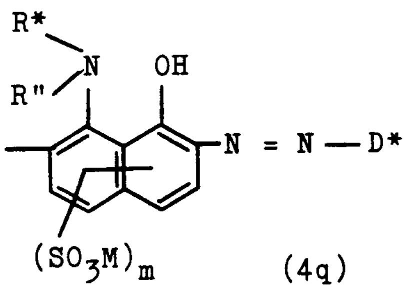
(4q)

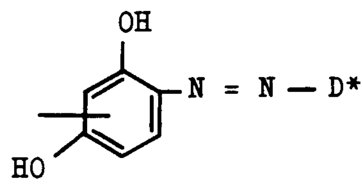
(4r)

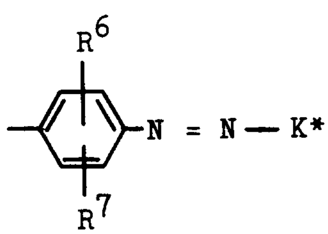
(4s)

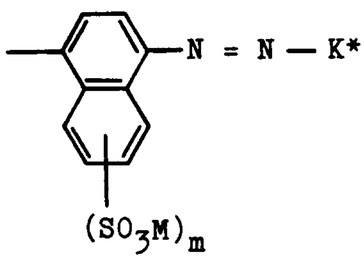
(4t)

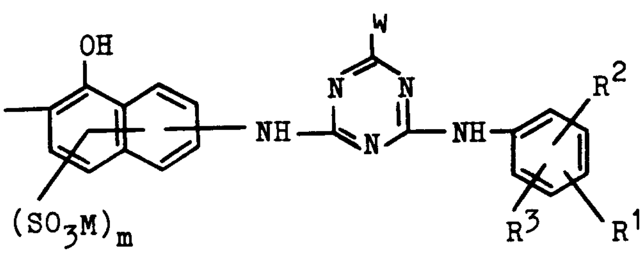
(4v)

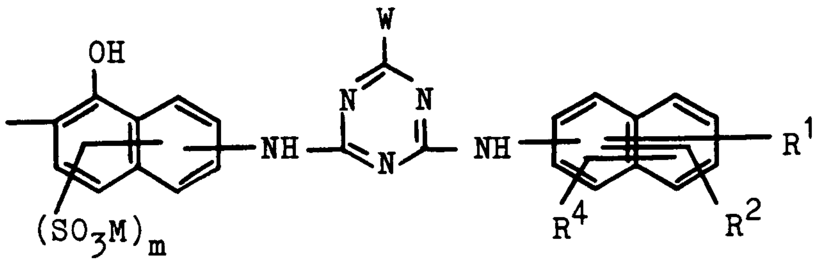

(4w)

In diesen Formeln bedeuten:

- $R^1$ ist ein Wasserstoffatom oder eine Carboxy- oder Sulfogruppe oder eine Gruppe der allgemeinen Formel $-SO_2-Y$ mit Y der obigen Bedeutung;
- $R^2$ ist ein Wasserstoffatom, eine Alkylgruppe von 1 bis 4 C-Atomen, wie insbesondere eine Methyl- oder Ethylgruppe, eine Alkoxygruppe von 1 bis 4 C-Atomen, wie insbesondere eine Methoxy- oder Ethoxygruppe, ein Chlor- oder Bromatom oder eine Carboxy-, Sulfo- oder Nitrogruppe;
- $R^3$ ist ein Wasserstoffatom, eine Alkylgruppe von 1 bis 4 C-Atomen, wie insbesondere die Methyl- oder Ethylgruppe, eine Alkoxygruppe von 1 bis 4 C-Atomen, wie insbesondere die Methoxy- oder Ethoxygruppe, ein Chlor- oder Bromatom;
- $R^4$ ist ein Wasserstoffatom, eine Sulfo- oder Carboxygruppe, bevorzugt ein Wasserstoffatom, falls $R^1$ eine Gruppe $-SO_2-Y$ ist;
- $B^1$ ist eine Alkylgruppe von 1 bis 4 C-Atomen, wie insbesondere die Methylgruppe, eine Carboxygruppe, eine Carbalkoxygruppe von 2 bis 5 C-Atomen, die Carbamoylgruppe oder ein gegebenenfalls durch Sulfo, Carboxy, Methyl, Ethyl, Methoxy, Ethoxy und/oder Chlor substituierter Phenylrest;
- $B^2$ ist eine Alkylgruppe von 1 bis 4 C-Atomen, wie insbesondere die Methylgruppe, eine Carboxygruppe, eine Carbalkoxygruppe von 2 bis 5 C-Atomen, die Carbamoylgruppe oder ein Phenylrest, der durch 1 oder 2 Substituenten aus der Gruppe Alkyl von 1 bis 4 C-Atomen, Alkoxy von 1 bis 4 C-Atomen, Chlor, Brom und Sulfo substituiert sein kann;
- Q ist ein Phenylrest, der substituiert sein kann, wie beispielsweise durch 1, 2 oder 3, bevorzugt 1 oder 2, Substituenten aus der Gruppe Chlor, Brom, Methyl, Ethyl, Methoxy, Ethoxy, Carboxy, Sulfo und Alkanoylamino, wie Acetylamino, und/oder durch eine Gruppe der allgemeinen Formel $-SO_2-Y$ mit Y der obengenannten Bedeutung, oder ist ein Naphthylrest, der durch 1, 2 oder 3 Sulfogruppen und gegebenenfalls durch eine Alkylgruppe von 1 bis 4 C-Atomen, eine Alkoxygruppe von 1 bis 4 C-Atomen, ein Chloratom oder eine Alkanoylaminogruppe von 2 bis 5 C-Atomen und/oder durch eine Gruppe der allgemeinen Formel $-SO_2-Y$ mit Y der obengenannten Bedeutung substituiert sein kann;
- $R^*$ ist ein Wasserstoffatom oder eine Alkylgruppe von 1 bis 4 C-Atomen, die durch einen Phenylrest oder eine durch Sulfo und/oder $-SO_2-Y$ mit Y der obigen Bedeutung substituierten Phenylrest substituiert sein kann;
- $R''$ ist ein Wasserstoffatom, eine Alkylgruppe von 1 bis 4 C-Atomen, die durch einen Phenylrest oder eine Gruppe der Formel $-SO_2-Y$ mit Y der obigen Bedeutung substituiert sein kann, oder ist ein Phenylrest, der durch 1 oder 2 Substituenten aus der Gruppe Alkyl von 1 bis 4 C-Atomen, Alkoxy von 1 bis 4 C-Atomen, Chlor, Brom, Sulfo und $-SO_2-Y$ mit Y der obigen Bedeutung substituiert sein kann;
- $R^5$ ist die Phenylureidogruppe, deren Phenylrest durch eine Gruppe der Formel $-SO_2Y$ mit Y der obigen Bedeutung substituiert sein kann, oder ist eine Alkanoylaminogruppe von 2 bis 5 C-Atomen, wie die Acetylamino- oder Propionylaminogruppe, die im Alkylrest durch eine Gruppe der Formel $-SO_2-Y$ mit Y der obigen Bedeutung substituiert sein kann, oder ist eine Alkenoylaminogruppe von 3 bis 5 C-Atomen, wie die Acryloylaminogruppe, oder ist eine Benzoylaminogruppe, die durch Substituenten aus der Gruppe Chlor, Methyl, Methoxy, Nitro, Sulfo, Carboxy und $-SO_2-Y$ mit Y der obigen Bedeutung substituiert sein kann, und ist bevorzugt der Acetylamino- oder Benzoylamino-Rest;
- $R^6$ ist ein Wasserstoffatom oder eine Alkylgruppe von 1 bis 4 C-Atomen, eine Alkoxygruppe von 1 bis 4 C-Atomen, eine Sulfogruppe, eine Carboxygruppe, eine Carbalkoxygruppe von 2 bis 5 C-Atomen, ein Halogenatom, wie Brom- oder Chloratom, oder eine Alkoxygruppe von 1 bis 4 C-Atomen, die durch eine Hydroxy-, Acetyloxy-, Carboxy-, Carbamoyl-oder

Cyangruppe oder ein Halogenatom, wie Chloratom, substituiert ist;

$R^7$ ist ein Wasserstoffatom, eine Alkylgruppe von 1 bis 4 C-Atomen, eine Alkoxygruppe von 1 bis 4 C-Atomen, ein Halogenatom, wie Brom- oder Chloratom, die Cyangruppe, die Trifluormethylgruppe, eine Alkoxygruppe von 1 bis 4 C-Atomen, die durch eine Hydroxy-, Acetyloxy-, Carboxy-, Carbamoyl- oder Cyangruppe oder durch ein Halogenatom, wie Chloratom, oder eine Gruppe der Formel $-SO_2-Y$ mit Y der obigen Bedeutung substituiert ist, oder ist eine Alkanoylaminogruppe von 2 bis 5 C-Atomen, die durch Chlor, Brom, Alkoxy von 1 bis 4 C-Atomen, Phenoxy, Phenyl, Hydroxy, Carboxy oder Sulfo oder eine Gruppe der Formel $-SO_2-Y$ mit Y der obigen Bedeutung substituiert sein kann, oder ist eine Alkenoylaminogruppe von 3 bis 5 C-Atomen, die durch Chlor, Brom, Carboxy oder Sulfo substituiert sein kann, oder ist die Benzoylaminogruppe, die im Benzolkern substituiert sein kann, beispielsweise durch Substituenten aus der Gruppe Chlor, Methyl, Sulfo und eine Gruppe der Formel $-SO_2-Y$ mit Y der obigen Bedeutung, oder ist eine Alkylsulfonylgruppe von 1 bis 4 C-Atomen oder die Phenylsulfonylgruppe, die im Benzolkern substituiert sein kann, beispielsweise durch Substituenten aus der Gruppe Chlor, Methyl, Sulfo und eine Gruppe der Formel $-SO_2-Y$ mit Y der obigen Bedeutung, oder ist eine Alkylsulfonylaminogruppe von 1 bis 4 C-Atomen, die durch Hydroxy, Sulfato, Chlor, Brom, Alkoxy von 1 bis 4 C-Atomen oder eine Gruppe der Formel $-SO_2-Y$ mit Y der obigen Bedeutung substituiert sein kann, oder ist die Phenylsulfonylaminogruppe, die im Benzolkern substituiert sein kann, beispielsweise durch Substituenten aus der Gruppe Chlor, Methyl, Sulfo und eine Gruppe der Formel $-SO_2-Y$ mit Y der obigen Bedeutung, oder ist die Carbamoylgruppe, die am Stickstoffatom durch 1 oder 2 Substituenten mono- oder disubstituiert sein kann, wobei die Substituenten der Gruppe Alkyl von 1 bis 4 C-Atomen, durch beispielsweise Hydroxy, Sulfo, Carboxy, Sulfato, Phenyl oder eine Gruppe der Formel $-SO_2-Y$ mit Y der obigen Bedeutung substituiertes Alkyl von 1 bis 4 C-Atomen, Cycloalkyl mit 5 bis 8 C-Atomen, Phenyl und durch Substituenten, beispielsweise aus der Gruppe Chlor, Sulfo, Methyl, Methoxy, Carboxy und eine Gruppe der Formel $-SO_2-Y$ mit Y der obigen Bedeutung, substituiertes Phenyl angehören, oder ist die Sulfamoylgruppe, die am Stickstoffatom durch 1 oder 2 Substituenten mono- oder disubstituiert sein kann, wobei die Substituenten der Gruppe Alkyl von 1 bis 4 C-Atomen, durch Substituenten, beispielsweise durch Hydroxy, Sulfo, Carboxy, Sulfato, Phenyl oder eine Gruppe der Formel $-SO_2-Y$ mit Y der obigen Bedeutung, substituiertes Alkyl von 1 bis 4 C-Atomen, Cycloalkyl mit 5 bis 8 C-Atomen, Phenyl und durch Substituenten, beispielsweise aus der Gruppe Chlor, Sulfo, Methyl, Methoxy, Carboxy und eine Gruppe der Formel $-SO_2-Y$ mit Y der obigen Bedeutung, substituiertes Phenyl angehören, oder ist die Ureidogruppe oder eine Ureidogruppe, die am endständigen Stickstoffatom durch 1 oder 2 Substituenten mono- oder disubstituiert sein kann, wobei die Substituenten der Gruppe Alkyl von 1 bis 4 C-Atomen, durch Substituenten, beispielsweise durch Hydroxy, Sulfo, Carboxy, Sulfato, Phenyl oder eine Gruppe der Formel $-SO_2-Y$ mit Y der obigen Bedeutung, substituiertes Alkyl von 1 bis 4 C-Atomen, Cycloalkyl mit 5 bis 8 C-Atomen, Phenyl und durch Substituenten, beispielsweise aus der Gruppe Chlor, Sulfo, Methyl, Methoxy, Carboxy und eine Gruppe der Formel $-SO_2-Y$ mit Y der obigen Bedeutung, substituiertes Phenyl angehören;

$R^8$ ist ein Wasserstoffatom oder eine Alkylgruppe von 1 bis 4 C-Atomen, die durch beispielsweise Hydroxy, Sulfo, Carboxy, Sulfato, eine Gruppe $-SO_2-Y$ mit Y der obigen Bedeutung oder Phenyl substituiert sein kann, oder ist eine Alkenylgruppe von 2 bis 4 C-Atomen, die durch eine Carboxy- oder Sulfogruppe oder durch ein Chlor- oder Bromatom substituiert sein kann, oder ist ein Cycloalkylrest von 5 bis 8 C-Atomen;

$R^9$ ist ein Wasserstoffatom oder eine Alkylgruppe von 1 bis 4 C-Atomen, die substituiert sein kann, beispielsweise durch Hydroxy, Sulfo, Carboxy, Sulfato, Phenyl oder $-SO_2-Y$ mit Y obiger Bedeutung, oder ist eine Alkenylgruppe von 2 bis 5 C-Atomen, die durch eine Carboxy- oder Sulfogruppe oder $-SO_2-Y$ mit Y obiger Bedeutung oder durch ein Chlor- oder Bromatom substituiert sein kann, oder $R^9$ ist ein Cycloalkylrest von 5 bis 8 C-Atomen oder ein Phenylrest, der substituiert sein kann, beispielsweise durch Substituenten aus der Gruppe Chlor, Sulfo, Methyl, Methoxy, Carboxy und $-SO_2-Y$ mit Y obiger Bedeutung, oder

$R^8$ und $R^9$ stellen zusammen mit dem Stickstoffatom und gegebenenfalls einem weiteren Heteroatom einen gesättigten heterocyclischen Rest dar, wie beispielsweise den Piperidino-, Morpholino- oder Piperazinorest;

$R^{10}$ ist ein Wasserstoffatom oder eine Alkylgruppe von 1 bis 4 C-Atomen oder eine durch Alkoxy von 1 bis 4 C-Atomen oder Cyan substituierte Alkylgruppe von 1 bis 4 C-Atomen;

$R^{11}$ ist ein Wasserstoffatom oder eine Sulfogruppe oder eine Sulfoalkylgruppe mit einem Alkylenrest von 1 bis 4 C-Atomen, wie Sulfomethylengruppe, oder eine Cyan- oder Carbamoylgruppe;

$B^3$ ist ein Wasserstoffatom oder eine Alkylgruppe von 1 bis 6 C-Atomen, bevorzugt von 1 bis 4 C-Atomen, die durch Phenyl, Sulfo, Sulfophenyl oder $-SO_2-Y$ mit Y obiger Bedeutung substituiert sein kann;

$B^4$ ist ein Wasserstoffatom, eine Alkylgruppe von 1 bis 4 C-Atomen, eine durch eine Alkoxygruppe von 1 bis 4 C-Atomen, wie Methoxygruppe, oder eine durch eine Sulfo-, Carboxy-, Sulfato-, Acetylamino-, Benzoylamino- oder Cyangruppe oder eine Gruppe der Formel $-SO_2-Y$ mit Y obiger Bedeutung substituierte Alkylgruppe von 1 bis 4 C-Atomen oder ist eine Alkenylgruppe von 2 bis 4 C-Atomen, die Cyclohexylgruppe, die Phenylgruppe oder eine durch Substituenten aus der Gruppe Carboxy, Sulfo, Benzoylamino, Acetylamino, $-SO_2-Y$ mit Y obiger Bedeutung und Chlor substituierte Phenylgruppe;

k ist die Zahl Null oder 1 (wobei im Falle k = Null diese Gruppe für ein Wasserstoffatom steht);

m steht für die Zahl 1 oder 2;

$m_1$ steht für die Zahl 1, 2 oder 3;

D* hat eine der für die allgemeine Formel (2) angegebenen Bedeutungen, hierbei bevorzugt die gleiche Bedeutung, oder ist ein Phenylrest, der durch 1, 2 oder 3, bevorzugt 1 oder 2, Substituenten aus der Gruppe Alkyl von 1 bis 4 C-Atomen, Alkoxy von 1 bis 4 C-Atomen, Chlor, Brom, Hydroxy, Carboxy, Sulfo, Carbamoyl, Sulfamoyl und Alkanoylamino, bevorzugt hiervon Methyl, Methoxy, Ethoxy, Chlor, Sulfo, Carboxy und Hydroxy, und/oder durch eine Gruppe der Formel $-SO_2-Y$ mit Y der obengenannten Bedeutung substituiert sein kann, wobei bevorzugt einer dieser Substituenten eine Sulfo- oder Carboxygruppe ist und die Gruppe $-SO_2-Y$ bevorzugt in meta- oder para-Stellung zur Azogruppe steht, oder ist ein Naphthylrest, der durch 1, 2 oder 3 Sulfogruppen oder durch 1 oder 2 Sulfogruppen und 1 oder 2 Gruppen der allgemeinen Formel $-SO_2-Y$ mit Y der obengenannten Bedeutung oder nur durch eine solche Gruppe $-SO_2-Y$ substituiert ist,

wobei D und D* zueinander gleiche oder voneinander verschiedene Bedeutungen besitzen können;

K* ist ein Rest aus einer der oben genannten und definierten allgemeinen Formeln (4a) bis (4m), wobei K und K* zueinander gleiche oder voneinander verschiedene Bedeutungen besitzen können;

W ist eine Sulfogruppe oder eine Alkylsulfonylgruppe von 1 bis 4 C-Atomen oder die Phenylsulfonylgruppe oder ein Bromatom oder bevorzugt ein Fluor- oder Chloratom;

M hat eine der oben genannten Bedeutungen.

Die einzelnen Formelglieder können zueinander gleiche oder voneinander verschiedene Bedeutungen besitzen.

Die in den obigen Formeln (4e), (4f), (4g), (4h), (4i) und (4n) befindlichen freien Bindungen, welche zur Azogruppe führen, bzw. die Azogruppe in Formel (4p) und (4q) befinden sich in ortho-Stellung zur Hydroxy- bzw. Aminogruppe gebunden. Bevorzugt steht diese Hydroxygruppe in $\alpha$-Stellung an den Naphthalinrest gebunden.

Alkylgruppen von 1 bis 4 C-Atomen sind bevorzugt die Ethyl-und insbesondere die Methylgruppe; Alkoxygruppen von 1 bis 4 C-Atomen sind bevorzugt die Ethoxy- und insbesondere die Methoxygruppe; Alkanoylaminogruppen von 2 bis 5 C-Atomen sind bevorzugt die Propionylaminogruppe und insbesondere die Acetylaminogruppe und Carbalkoxygruppen von 2 bis 5 C-Atomen, bevorzugt die Carbomethoxy- und Carbethoxygruppe.

Von den erfindungsgemäßen Verbindungen der allgemeinen Formel (1) sind insbesondere diejenigen bevorzugt, in welchen K einen Rest der allgemeinen Formel (4c), (4f), (4h), (4p) oder (4q) bedeutet, in welchen wiederum die einzelnen Formelglieder die folgenden bevorzugten Bedeutungen besitzen:

$B^1$ ist eine Carboxy- oder Methylgruppe;

Q ist ein Phenylrest, der durch 1 oder 2 Substituenten substituiert sein kann, die aus der folgenden Menge an Substituenten ausgewählt sind: 2 Methyl, 2 Methoxy, 1 Chlor oder Brom, 2 Sulfo, 1 Carboxy und 1 Vinylsulfonyl oder $\beta$-Sulfatoethylsulfonyl;

$R^5$ ist die Acetylamino- oder Propionylaminogruppe oder eine Benzoylaminogruppe, die durch 1 oder 2 Substituenten aus der Gruppe Chlor, Methyl, Methoxy, Nitro, Sulfo oder $\beta$-Sulfatoethylsulfonyl substituiert sein kann;

R* und R″ sind beide ein Wasserstoffatom.

Insbesondere bevorzugt sind solche Verbindungen der allgemeinen Formel (1), in welchen K den durch 1, 2 oder 3 Sulfogruppen substituierten 1-Hydroxy-naphth-2-yl-Rest darstellt oder einen Rest der allgemeinen Formel (4c) bedeutet, in welcher $B^1$ eine Carboxy- oder Methylgruppe ist und Q für einen Phenylrest steht, der durch 1 oder 2 Substituenten substituiert ist, die aus der Gruppe von 2 Methylgruppen, 2 Ethoxygruppen, 2 Methoxygruppen, 2 Sulfogruppen, 1 Carboxygruppe und 1 Chloratom ausgewählt sind, wobei einer der Substituenten zwingend eine Carboxy-oder Sulfogruppe ist, oder der Phenylrest durch eine Vinylsulfonyl- oder β-Sulfatoethylsulfonyl-Gruppe substituiert ist und zusätzlich durch 1 oder 2 Substituenten substituiert sein kann, die aus der Gruppe von 1 Methyl, 2 Methoxy, 1 Chlor und 1 Sulfo ausgewählt sind.

In der Komponente K der allgemeinen Formel (4v) ist $R^1$ bevorzugt eine Gruppe $-SO_2-Y$ mit Y der obengenannten, insbesondere bevorzugten Bedeutung, $R^2$ ein Wasserstoffatom, eine Alkylgruppe von 1 bis 4 C-Atomen, eine Alkoxygruppe von 1 bis 4 C-Atomen, ein Chlor- oder Bromatom oder eine Carboxy-, Sulfo- oder Nitrogruppe und $R^3$ ein Wasserstoffatom.

Die vorliegende Erfindung betrifft weiterhin Verfahren zur Herstellung der erfindungsgemäßen Azoverbindungen der allgemeinen Formel (1), beispielsweise durch Kupplungsreaktion der Diazoniumverbindung einer Aminoverbindung der allgemeinen Formel (5)

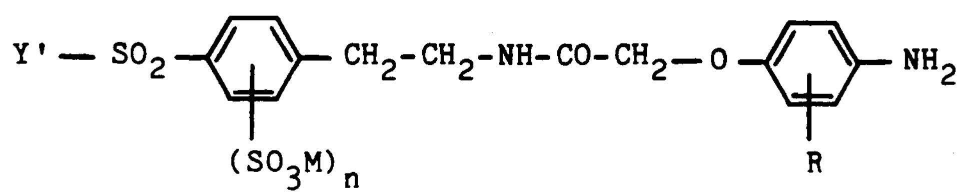

(5)

in welcher Y′ eine der Bedeutungen von Y hat oder die β-Hydroxyethyl-Gruppe ist und R, M und n die obengenannten Bedeutungen haben, mit einer Kupplungskomponente der allgemeinen Formel H-K mit K der obengenannten Bedeutung; sofern K, wie oben angegeben, eine bivalente Kupplungskomponente ist, kann eine Disazoverbindung, sofern diese erwünscht ist, durch Umsetzung dieser bivalenten Kupplungskomponente mit der zweifach äquimolaren Menge der Diazokomponente hergestellt werden. Im Falle der Verwendung einer Verbindung (5) mit Y′ gleich einer β-Hydroxyethyl-Gruppe wird diese β-Hydroxyethylgruppe in der gebildeten Azoverbindung in eine Gruppe Y der erfindungsgemäßen Azoverbindung (1), wie später noch angegeben, übergeführt.

Die Diazotierungs- und Kupplungsreaktionen erfolgen in üblicher und altbekannter Weise, so die Diazotierung des Amins (5) in der Regel bei einer Temperatur zwischen -5 °C und +15 °C und einem pH-Wert unterhalb von 2 mittels einer starken Säure und Alkalinitrit in bevorzugt wäßrigem Medium und die Kupplungsreaktion in der Regel bei einem pH-Wert zwischen 1,5 und 4,5 im Falle einer aminogruppenhaltigen Kupplungskomponente und bei einem pH-Wert zwischen 3 und 7,5 im Falle einer hydroxygruppenhaltigen Kupplungskomponente und bei einer Temperatur zwischen 0 und 30 °C in bevorzugt wäßrigem Medium.

Ist die Kupplungskomponente eine bivalente, doppelankuppelbare Verbindung, enthält sie beispielsweise eine kupplungsfähige Aminogruppe und gleichzeitig eine kupplungsfähige Hydroxygruppe, so kann zur Herstellung einer Disazoverbindung die Kupplung zunächst mit dem ersten Mol der Diazoniumverbindung des Amins im sauren pH-Bereich zur Monoazoverbindung erfolgen und die zweite Kupplungsreaktion mit dem zweiten Mol der Diazoniumverbindung des Amins anschließend im schwach sauren bis schwach alkalischen Bereich. Diese Verfahrensweise gilt beispielsweise für die Verbindungen entsprechend den allgemeinen Formeln (4p) und (4q), so durch Kupplung der Aminonaphtholsulfonsäure zunächst mit dem ersten Mol der Diazoniumverbindung des Amins der allgemeinen Formel (5) oder eines anderen aromatischen Amins entsprechend der allgemeinen Formel $D^*-NH_2$ mit $D^*$ der obengenannten anderen Bedeutung als D im sauren Medium und sodann durch Kupplung der gebildeten Monoazoverbindung mit dem zweiten Mol einer Diazoniumverbindung eines Amins $D^*-NH_2$ mit $D^*$ der obengenannten Bedeutung im schwach sauren, neutralen oder schwach alkalischen Bereich, wobei $D^*$ zwingend eine der für D angegebenen Bedeutungen besitzt, sofern die erste Kupplungsreaktion nicht mit einer Diazoniumverbindung eines Amins (5) durchgeführt wurde, so insbesondere zunächst bei einem pH-Wert von etwa 1 bis 2,5 und anschließend bei einem pH-Wert zwischen 4 und 6,5, wobei, sofern die Diazoniumverbindung der Aminoverbindung (5) in beiden Kupplungsreaktionen identisch ist, die erste und zweite Kupplungsreaktion in ein und demselben

Ansatz, zunächst im angegebenen sauren Bereich und sodann im schwach sauren bis schwach alkalischen Bereich, durchgeführt werden kann. Zur Herstellung einer Disazoverbindung entsprechend der allgemeinen Formel (4r) erfolgt die Umsetzung der Kupplungskomponente Resorcin mit der bzw. den Diazoniumverbindung(en) vorteilhaft zunächst bei einem pH-Wert zwischen 0,8 und 2 und sodann bei einem pH-Wert zwischen 6 und 7,5.

Disazoverbindungen entsprechend der allgemeinen Formel (1), deren Rest K dem Rest einer Azoverbindung entspricht, welcher aus einer kupplungsfähigen Diazokomponente und einer Kupplungskomponente zusammengesetzt ist, wie beispielsweise der Rest entsprechend der allgemeinen Formel (4s) oder (4t), lassen sich auch erfindungsgemäß in der Weise herstellen, daß man zunächst die Diazoniumverbindung eines Amins (5) mit der aminogruppenhaltigen und somit diazotierbaren Kupplungskomponente, wie beispielsweise in den Formeln (4s) und (4t) die durch die Substituenten $R^6$ und $R^7$ substituierten Anilin- bzw. Sulfo-aminonaphthalin-Komponenten, kuppelt und in der so gebildeten Amino-Azoverbindung die Aminogruppe diazotiert und mit einer Kupplungskomponente, wie beispielsweise der Kupplungskomponente H-K* , zur Disazoverbindung kuppelt.

Alle diese Umsetzungsmöglichkeiten zur Synthese von Disazoverbindungen sind analog den in der Literatur bekannten oder dem Fachmann geläufigen Methoden zur Synthese von Disazoverbindungen.

Kupplungskomponenten, die zur Herstellung der erfindungsgemäßen Farbstoffe verwendet werden können und beispielsweise den allgemeinen Formeln (4a) bis (4n) entsprechen, sind beispielsweise:
1,3-Diamino-benzol-5-sulfonsäure, Phenol, Kresol, Resorcin, 2-Ethoxy-phenol, 4-Methylphenol, 3-Sulfophenol, Salicylsäure, 3-Sulfo-1-naphthol, 4-Sulfo-1-naphthol, 5-Sulfo-1-naphthol, 3,6-Disulfo-8-naphthol, 4,6-Disulfo-8-naphthol,1-Naphthol-3,8-disulfonsäure,
1-Amino-8-naphthol-4-sulfonsäure, 1-Amino-8-naphthol-5-sulfonsäure, 1-Amino-8-naphthol-2,4-disulfonsäure, 2-Amino-5-naphthol-7-sulfonsäure, 2-Amino-5-naphthol-1,7-disulfonsäure, 1-Amino-5-naphthol-7-sulfonsäure, 2-Amino-8-naphthol-6-sulfonsäure, 2-Amino-8-naphthol-3,6-disulfonsäure, 2-Amino-8-naphthol-4,6-disulfonsäure,
1-Amino-8-naphthol-3,6- oder -4,6-disulfonsäure, 1-Acryloylamino-8-naphthol-3,6- oder -4,6-disulfonsäure, 1-Propionylamino-8-naphthol-3,6- oder -4,6-disulfonsäure,
1-Acetylamino-8-naphthol-4-sulfonsäure, 1-Acetylamino-8-naphthol-3,6- oder -4,6-disulfonsäure, 1-Benzoylamino-8-naphthol-3,6- oder -4,6-disulfonsäure, 2-Naphthol-5,7-disulfonsäure, 2-Naphthol-3,6- und -6,8-disulfonsäure, 1,8-Dihydroxynaphthalin-3,6-disulfonsäure,
1,8-Dihydroxynaphthalin-6-sulfonsäure, 1-Naphthol-3,6,8-trisulfonsäure,2-Acetylamino-5-naphthol-7-sulfonsäure, 2-Benzoylamino-8-naphthol-6-sulfonsäure,
2-(p′-Tosylamino)-5-naphthol-7-sulfonsäure, 2-Acetylamino-8-naphthol-3,6-disulfonsäure, 2-Acetylamino-5-naphthol-1,7-disulfonsäure, 3-Benzoylamino-8-naphthol-6-sulfonsäure, 2-Phenylsulfonylamino-5-naphthol-7-sulfonsäure, 2-(N-Methyl-N-acetyl)-amino-8-naphthol-6-sulfonsäure, N-Ethyl-N-benzylanilin-3-sulfonsäure, N,N-Bis-(β-hydroxyethyl)-anilin,
N,N-Bis-(β-sulfatoethyl)-anilin, N,N-Bis-(β-hydroxyethyl)-2-methoxy-5-chlor-anilin, N-(β-Sulfatoethyl)-2,5-dimethoxyanilin, N-(β-Sulfatoethyl)-2-chloranilin, Acetoacetyl-2-naphthylamid-5-sulfonsäure, N-Acetoacetylanilin-3- oder -4-sulfonsäure, N-Acetoacetyl-2-methoxy-5-sulfo-anilin, N-Acetoacetyl-4-methoxy-3-sulfoanilin, N-Acetoacetyl-2-methoxy-5-methyl-4-sulfo-anilin, N-Acetoacetyl-2,5-dimethoxy-4-sulfo-anilin, N-Acetoacetyl-2-methoxy-5-methyl-4-(β-sulfatoethylsulfonyl)-anilin,
N-Acetoacetyl-2,5-dimethoxy-4-(β-sulfatoethylsulfonyl)-anilin, N-Acetoacetyl-2-methoxy-5-(β-sulfatoethylsulfonyl)-anilin, N-Acetoacetyl-4-(β-sulfatoethylsulfonyl)-anilin, N-Acetoacetyl-3-(β-sulfatoethylsulfonyl)-anilin,
1-(4′-β-Sulfatoethylsulfonyl-phenyl)-3-methyl-pyrazolon(5), 1-(4′-β-Sulfatoethylsulfonyl-phenyl)-3-carboxy-pyrazolon(5), 1-(4′-Sulfophenyl)-3-methyl-pyrazolon(5), 1-(4′-Sulfophenyl)-3-carboxy-pyrazolon(5), 1-(2′-Chlor-5′sulfo-phenyl)-3-methyl-oder -3-carboxy-pyrazolon(5), 1-(3′-Sulfophenyl)-3-carboxypyrazolon(5), 1-(2′-Methoxy-4′-sulfophenyl)-3-carboxy-pyrazolon(5), 1-(3′-Sulfophenyl)-3-methyl-5-amino-pyrazol, 1-(4′-Sulfophenyl)-3-methyl-5-amino-pyrazol, 1-(2′-Methoxy-5′-sulfo-phenyl)-3-methyl-5-aminopyrazol, 1-(2′-Methoxy-5′-methyl-4′-sulfophenyl)-3-methyl-5-aminopyrazol,
1-(2′-Chlor-5′-sulfo-phenyl)-3-methyl-5-aminopyrazol, 1-(3′-Amino-4′-sulfo-phenyl)-3-carbethoxy-pyrazolon(5), 1-(4′-β-Sulfatoethylsulfonyl-phenyl)-3-carbethoxy-pyrazolon(5), 1-(3′-Amino-6′methyl-phenyl)-3-carboxy-pyrazolon-(5),
2-N-Methylamino-8-naphthol-6-sulfonsäure, 3-Carboxy-pyrazolon(5),1-Phenyl-3-carboxy-pyrazolon(5),
1-(4′-Nitrophenyl)-3-carboxypyrazolon(5),
1-(3′-Acetylaminophenyl)-3-carboxy-pyrazolon(5), 1-(3′-Carboxyphenyl)-3-methyl-pyrazolon(5), 2-Hydroxy-3-carboxy-naphthalin, 2-Hydroxy-6-carboxy-naphthalin, 8-Hydroxy-chinolin-5-sulfonsäure, 1,4-Dimethyl-2-hydroxy-6-pyridon-5-sulfonsäure, N-Sulfomethyl-anilin, 3-Acetylamino-5-naphthol-7-sulfonsäure, 2-Methylamino-8-naphthol-6-sulfonsäure, 2,5-Disulfo-diphenylamin, 4-Sulfo-diphenylamin, 1-[4′-Chlor-6′-(4″-β-sulfatoethylsulfonyl-phenyl)-amino-1′,3′,5′-triazin-2′-yl]-amino-8-naphthol-3,6-disulfonsäure, 1-[4′-Chlor-6′-(4″-β-sulfatoethylsulfonyl-phenyl)-amino-1′,3′,5′-triazin-2′-yl]-amino-8-naphthol-4,6-disulfonsäure, 2-[4′-Chlor-6′-(4″-β-sulfatoethylsulfonyl-phenyl)-amino-1′,3′,5′-triazin-2′-yl]-amino-8-naphthol-6-sulfonsäure, 3-[4′-Chlor-6′-(4″-β-sulfatoethylsulfonyl-phenyl)-amino-1′,3′,5′-triazin-2′-yl]-amino-8-naphthol-3,6-disulfonsäure, 1-(4′-Chlor-6′-methoxy-1′,3′,5′-triazin-2′-yl)-amino-8-naphthol-3,6-disulfonsäure, 1-(4′-Chlor-6′-methoxy-1′,3′,5′-triazin-2′-yl)-amino-8-naphthol-4,6-disulfonsäure,

2-(4′-Chlor-6′-methoxy-1′,3′,5′-triazin-2′-yl)-amino-8-naphthol-6-sulfonsäure, 3-(4′-Chlor-6′-methoxy-1′,3′,5′-triazin-2′-yl)-amino-8-naphthol-6-sulfonsäure, 1-[4′-Fluor-6′-(4″-β-Sulfatoethylsulfonyl-phenyl)-amino-1′,3′,5′-triazin-2′-yl]-amino-8-naphthol-3,6-disulfonsäure, 1-[4′-Fluor-6′-(4″-β-Sulfatoethylsulfonyl-phenyl)-amino-1′,3′,5′-triazin-2′-yl]-amino-8-naphthol-4,6-disulfonsäure, 2-[4′-Fluor-6′-(4″-β-Sulfatoethylsulfonyl-phenyl)-amino-1′,3′,5′-triazin-2′-yl]-amino-8-naphthol-6-sulfonsäure, 3-[4′-Fluor-6′-(4″-β-Sulfatoethylsulfonyl-phenyl)-amino-1′,3′,5′-triazin-2′-yl]-amino-8-naphthol-6-sulfonsäure, 1-(4′-β-Sulfatoethylsulfonyl-benzoyl)-amino-8-naphthol-3,6-disulfonsäure oder -4,6-disulfonsäure, 2- oder 3-(4′-β-Sulfatoethylsulfonyl-benzoyl)-amino-8-naphthol-6-sulfonsäure, 1-{4′-Chlor-6′-[β-(4″-β″-sulfatoethylsulfonyl-phenyl)-ethyl]-1′,3′,5′-triazin-2′-yl}-amino-8-naphthol-3,6- oder -4,6-disulfonsäure,

1-{4′-Chlor-6′-[β-(3″-β″-sulfatoethylsulfonyl-phenyl)-ethyl]-1′,3′,5′-triazin-2′-yl}-amino-8-naphthol-3,6- oder -4,6-disulfonsäure, 1-{4′-Chlor-6′-[β-(4″-sulfo-phenyl)-ethyl]-1′,3′,5′-triazin-2′-yl}-amino-8-naphthol-3,6- oder -4,6-disulfonsäure, 1-{4′-Chlor-6′-[β-(2″,5″-disulfophenyl)-ethyl]-1′,3′,5′-triazin-2′-yl}-amino-8-naphthol-3,6- oder -4,6-disulfonsäure, 1-{4′-Fluor-6′-[β-(3″,5″-disulfophenyl)-ethyl]-1′,3′,5′-triazin-2′-yl}-amino-8-naphthol-3,6- oder -4,6-disulfonsäure,

1-(β-Hydroxyethyl)-4-methyl-6-hydroxy-2-pyridon, 1-(β-Hydroxyethyl)-3-cyano-4-methyl-6-hydroxy-2-pyridon, 1-(β-Hydroxyethyl)-3-carbamoyl-4-methyl-6-hydroxy-2-pyridon, 1-(β-Hydroxyethyl)-4-methyl-6-hydroxy-2-pyridon-3-sulfonsäure, 1-(β-Sulfatoethyl)-4-methyl-6-hydroxy-2-pyridon, 1-(β-Sulfatoethyl)-3-cyano-4-methyl-6-hydroxy-2-pyridon, 1-(β-Sulfatoethyl)-3-carbamoyl-4-methyl-6-hydroxy-2-pyridon, 1-(β-Sulfatoethyl)-4-methyl-6-hydroxy-2-pyridon-3-sulfonsäure, 1-(β-Sulfatoethyl)-4-methyl-6-hydroxy-2-pyridon, 1-(β-Sulfatoethyl)-3-carbamoyl-4-methyl-6-hydroxy-2-pyridon, 1-(β-Sulfatoethyl)-4-methyl-6-hydroxy-2-pyridon-3-sulfonsäure, 1-Carboxymethyl-4-methyl-6-hydroxy-2-pyridon, 1-Carboxymethyl-3-cyano-4-methyl-6-hydroxy-2-pyridon, 1-Carboxymethyl-3-carbamoyl-4-methyl-6-hydroxy-2-pyridon, 1-Carboxymethyl-4-methyl-6-hydroxy-2-pyridon-3-sulfonsäure, 1-(β-Carboxyethyl)-4-methyl-6-hydroxy-2-pyridon, 1-(β-Carboxyethyl)-3-cyano-4-methyl-6-hydroxy-2-pyridon, 1-(β-Carboxyethyl)-3-carbamoyl-4-methyl-6-hydroxy-2-pyridon, 1-(β-Carboxyethyl)-4-methyl-6-hydroxy-2-pyridon-2-sulfonsäure, 1-(β-Acetylaminoethyl)-4-methyl-6-hydroxy-2-pyridon, 1-(β-Acetylaminoethyl)-3-cyano-4-methyl-6-hydroxy-2-pyridon, 1-(β-Acetylaminoethyl)-3-carbamoyl-4-methyl-6-hydroxy-2-pyridon, 1-(β-Acetylaminoethyl)-4-methyl-6-hydroxy-2-pyridon-3-sulfonsäure,

1-(β-Acetylaminopropyl)-4-methyl-6-hydroxy-2-pyridon, 1-(β-Acetylaminopropyl)-3-cyano-4-methyl-6-hydroxy-2-pyridon, 1-(β-Acetylamino-propyl)-3-carbamoyl-4-methyl-6-hydroxy-2-pyridon 1-(β-Acetylamino-propyl)-4-methyl-6-hydroxy-2-pyridon-3-sulfonsäure, 4-Hydroxy-chinolin-(2), 1-Amino-8-hydroxy-2-(phenylazo)-naphthalin-3,6-disulfonsäure, 1-Amino-8-hydroxy-2-(4′-sulfophenylazo)-naphthalin-3,6-disulfonsäure, 1-Amino-8-hydroxy-2-(2′,5′-disulfophenylazo)-naphthalin-3,6-disulfonsäure, 1-(β-Aminoethyl)-3-cyano-4-methyl-6-hydroxy-2-pyridon, 1-(q-Aminopropyl)-3-sulfomethyl-4-methyl-6-hydroxy-2-pyridon, 1,3-Diaminobenzol, 1-Amino-3-(N,N-di-β-hydroxyethylamino)-benzol, 1-Amino-3-(N,N-di-b-sulfatoethylamino)-benzol, 1-Amino(3-N,N-di-β-hydroxyethylamino)-4-methoxybenzol, 1-Amino-3-(N,N-di-β-sulfatoethylamino)-4-methoxy-benzol, 1-Amino-3-(sulfobenzylamino)-benzol, 1-Amino-3-(sulfobenzylamino)-4-chlorobenzol, 1-Amino-3-(N,N-di-sulfobenzylamino)-benzol, Phenol, 1-Hydroxy-3- oder -4-methyl-benzol, 1-Hydroxybenzol-4-sulfosäure, 1-Hydroxynaphthalin,

2-Hydroxynaphthalin, 2-Hydroxynaphthalin-6- oder -7-sulfonsäure, 1-Hydroxynaphthalin-4,7-disulfonsäure, 1-Amino-3-methyl-benzol, 1-Amino-2-methoxy-5-methyl-benzol, 1-Amino-2,5-dimethyl-benzol, 3-Aminophenylharnstoff, 1-Amino-3-acetylamino-benzol, 1-Amino-3-(hydroxyacetylamino)-benzol, 1,3-Diaminobenzol-4-sulfonsäure, 1-Amino-naphthalin-6- oder -8-sulfonsäure, 1-Amino-2-methoxy-naphthalin-6-sulfonsäure, 2-Amino-naphthalin-5,7-disulfonsäure, 1-Amino-8-hydroxy-naphthalin-6-sulfonsäure, 2-Hydroxy-3-aminonaphthalin-5,7-disulfonsäure, 1-Amino-8-hydroxy-naphthalin-2,4,6-trisulfonsäure, 1-Hydroxy-8-acetylamino-naphthalin-3-sulfonsäure, 1-Benzoylamino-8-hydroxy-naphthalin-3,6- oder -4,6-disulfonsäure, 2-Benzoylamino-5-hydroxy-naphthalin-7-sulfonsäure, 2-Methyl- und 2-Ethylamino-5-hydroxy-naphthalin-7-sulfonsäure, 2-(N-Acetyl-N-methylamino)-5-hydroxy-naphthalin-7-sulfonsäure, 2-Ethylamino-8-hydroxy-naphthalin-6-sulfonsäure, 2-Acetylamino-8-hydroxy-naphthalin-6-sulfonsäure, 1-(4′-Aminobenzoylamino)-8-hydroxynaphthalin-3,6- und -4,6-disulfonsäure, 1-(4′-Nitrobenzoylamino)-8-hydroxy-naphthalin-3,6 und

-4,6-disulfonsäure, 1-(3′-Amino-benzoylamino)-6-hydroxy-naphthalin-3,6- und -4,6-disulfonsäure, 1-(3′-Nitrobenzoylamino)-8-hydroxy-naphthalin-3,6- und -4,6-disulfonsäure, 2-(4′-Amino-3′-sulfophenyl)-amino-5-hydroxy-naphthalin-7-sulfonsäure, 3-Methyl-5-pyrazolon, 1-Phenyl-3-methyl-5-pyrazolon, 1-(3′-Aminophenyl)-3-methyl-5-pyrazolon, 1-(2',5'-Disulfophenyl)-3-methyl-5-pyrazolon, 1-(2'-Methyl-4'-sulfophenyl)-5-pyrazolon-3-carbonsäure, 1-(4',8'-Disulfonaphthyl-2'-yl)-3-methyl-5-pyrazolon, 1-(5',7'-Disulfonaphthyl-2-)-3-methyl-5-pyrazolon, 1-(2',5'-Dichlor-4'-sulfophenyl)-3-methyl-5-pyrazolon, 3-Aminocarbonyl-4-methyl-6-hydroxy-2-pyridon, 1-Ethyl-3-cyano- oder -3-chlor-4-methyl-6-hydroxy-2-pyridon, 1-Ethyl-3-sulfomethyl-4-methyl-6-hydroxy-2-pyridon, 2,4,6-Triamino-3-cyano-pyridin, 2-(3'-Sulfophenyl)-amino-4,6-diamino-3-cyano-pyridin, 2-(2'-Hydroxyethylamino)-3-cyano-4-methyl-6-amino-pyridin, 2,6-Bis-(2'-hydroxyethylamino)-3-cyano-4-methyl-pyridin, 1-Ethyl-3-carbamoyl-4-methyl-6-hydroxy-2-pyridon, 1-Ethyl-3-sulfomethyl-4-methyl-5-carbamoyl-6-hydroxy-2-pyridon,N-Acetoacetylamino-benzol.

Die erfindungsgemäß zur Synthese der erfindungsgemäßen Azoverbindungen (1) einsetzbaren Verbindungen entsprechend der allgemeinen Formel (5) sind bisher noch nicht bekannt. Die Erfindung betrifft somit auch diese Verbindungen, Verfahren zu deren Herstellung und ihre Verwendung zur Synthese von Farbstoffen, wie insbesondere zu den erfindungsgemäßen Azoverbindungen (1). Sie lassen sich analog bekannten Verfahrensweisen der Umsetzung von Säurechloriden mit Aminen herstellen, indem man zunächst eine Verbindung der allgemeinen Formel (6)

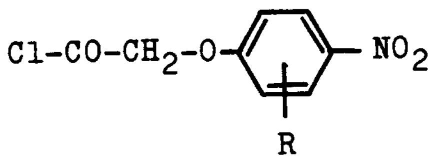

(6)

in welcher R die obengenannte Bedeutung hat, mit einer Aminoverbindung der allgemeinen Formel

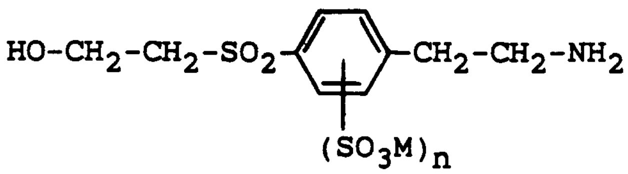

(7)

in welcher M und n die obengenannten Bedeutungen haben, umsetzt. Die Umsetzung erfolgt in hierfür üblichen und geeigneten Löse- oder Verdünnungsmitteln und in Gegenwart eines säurebindenden Mittels, in der Regel bei einer Temperatur zwischen -5 °C und +80 °C, bevorzugt zwischen 0 und 30 °C. Geeignete Lösemittel sind beispielsweise Wasser oder ein organisches Löse- oder
Verdünnungsmittel oder ein Gemisch von Wasser und einem Wasser mischbaren organischen Lösemittel. Organische Löse-oder Verdünnungsmittel sind beispielsweise Wasser, Alkanole von 1 bis 6 C-Atomen, bevorzugt 1 bis 4 C-Atomen, wie beispielsweise Methanol, Dioxan, Toluol, die Xylole, Chlorbenzol, o-Dichlorbenzol, m-Dichlorbenzol, Dimethylformamid und N-Methyl-pyrrolidon.
Säurebindende Mittel sind beispielsweise Kaliumcarbonat, Magnesiumoxid, Natriumcarbonat, Natriumhydroxid, Triethylamin und Triethanolamin. In wäßrigem Medium wird ein pH-Wert zwischen 6 und 12, bevorzugt zwischen 8 und 10, eingehalten.

Säurechloride der allgemeinen Formel (6) sind beispielsweise 4-Nitro-phenoxy-essigsäurechlorid, 2,4-Dinitro-phenoxy-essigsäurechlorid, 2-Chlor-4-nitro-phenoxy-essigsäurechlorid, 2-Brom-4-nitro-phenoxy-essigsäurechlorid, 2-Methyl-4-nitro-phenoxy-essigsäurechlorid und 2-Methoxy-4-nitro-phenoxy-essigsäurechlorid.

Aminoverbindungen der allgemeinen Formel (7) sind bspw. 4-($\beta$-Hydroxyethylsulfonyl)-1-($\beta$-aminoethyl)-benzol, 5-($\beta$-Hydroxyethylsulfonyl)-2-($\beta$-aminoethyl)-benzol-sulfonsäure.

Die auf diese Weise erfindungsgemäß erhältlichen und ebenfalls neuen, erfindungsgemäßen Carbonamid-Verbindungen entsprechend der allgemeinen Formel (8)

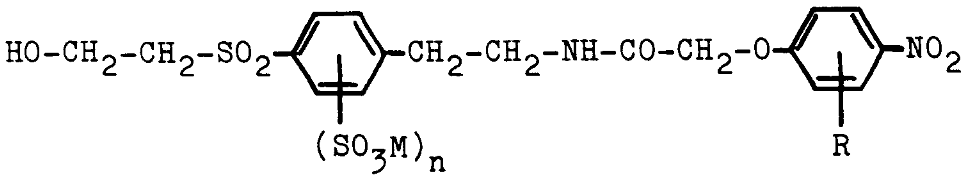

(8)

in welcher R, M und n die obengenannten Bedeutungen haben, werden sodann analog bekannten Verfahrensweisen, nachdem sie beispielsweise aus dem Reaktionsansatz durch Kristallisation oder durch Abdestillieren des Lösemittels oder durch Ansäuern und Filtration isoliert wurden, zur Aminoverbindung entsprechend der allgemeinen Formel (5) reduziert, so durch katalytische Hydrierung mit Wasserstoff an Palladium, Platin oder Raney-Nickel bei einer Temperatur zwischen 50 und 110 °C und bei erhöhtem Druck oder durch Reduktion nach Béchamp mit Eisen in saurem Medium, beispielsweise mit Eisen in Ethanol/Eisessig. Die Reduktion kann in einem hierfür geeigneten Lösemittel, wie Wasser, Methanol oder Ethanol oder einer Mischung derselben, erfolgen.

Sowohl die Verbindungen entsprechend der allgemeinen Formel (1) als auch die der allgemeinen Formel (5), in welchen Y bzw. Y′ die β-Hydroxyethyl-Gruppe bedeuten, können in üblicher und bekannter Verfahrensweise in Verbindungen übergeführt werden, in welchen Y bzw. Y′ eine andere Bedeutung als die β-Hydroxyethyl-Gruppe besitzt, so beispielsweise in deren Esterderivate, wie beispielsweise von mehrwertigen anorganischen Säuren oder von aliphatischen und aromatischen Carbon- oder Sulfonsäuren, so beispielsweise in Verbindungen, in welchen Y bzw. Y′ für die β-Chlorethyl-, β-Sulfatoethyl-, β-Phosphatoethyl-, β-Thiosulfatoethyl-, β-Acetyloxyethyl- oder β-Toluylsulfonyloxyethyl-Gruppe steht. Hierfür geeignete Veresterungs- und Acylierungsmittel sind beispielsweise die entsprechenden anorganischen oder organischen Säuren oder deren Anhydride oder Halogenide oder Amide, wie beispielsweise Schwefelsäure, Schwefeltrioxid enthaltende Schwefelsäure, Chlorsulfonsäure, Amidosulfonsäure, Phosphorsäure, Phosphoroxychlorid, Gemische aus Phosphorsäure und Phosphorpentoxid, Acetanhydrid, Toluolsulfochlorid und Thionylchlorid.

Diejenigen Verbindungen, in welchen Y bzw. Y′ für die Vinylgruppe steht, können aus deren analogen Esterderivaten mittels Alkali, so in wäßrigem Medium bei einem pH-Wert von 10 bis 12 und einer Temperatur zwischen 40 und 50 °C während 10 bis 20 Minuten, hergestellt werden. Die Synthese von beispielsweise β-(Dialkylamino)-ethylsulfonyl- und β-Thiosulfatoethylsulfonyl-Derivaten der Verbindungen (1) und (5) erfolgt durch Umsetzung von deren Vinylsulfonyl-Verbindungen mit dem entsprechenden Dialkylamin oder mit einem Alkalisalz der Thioschwefelsäure, wie Natriumthiosulfat.
Alle diese Verfahrensweisen der Überführung von einer Gruppe $-SO_2-Y$ bzw. $-SO_2-Y'$ in eine andere sind dem Fachmann auf diesem faserreaktiven Gebiet geläufig und zahlreich in der Literatur beschrieben.

Die erfindungsgemäßen Verbindungen der allgemeinen Formel (1) - im nachfolgenden als Verbindungen (1) bezeichnet - haben faserreaktive Eigenschaften und besitzen sehr wertvolle Farbstoffeigenschaften. Sie können deshalb zum Färben (einschließlich Bedrucken) von hydroxygruppenhaltigen und/oder carbonamidgruppenhaltigen Materialien verwendet werden. Hierzu können die bei der Synthese der Verbindungen (1) anfallenden Lösungen, gegebenenfalls nach Zusatz einer Puffersubstanz und gegebenenfalls auch nach Konzentrierung, direkt als Flüssigpräparation der färberischen Verwendung zugeführt werden.

Die Abscheidung und Isolierung der Verbindungen (1) aus den wäßrigen Syntheselösungen kann nach allgemein bekannten Methoden für wasserlösliche Verbindungen erfolgen, so beispielsweise durch Ausfällen aus dem Reaktionsmedium mittels eines Elektrolyten, wie beispielsweise Natriumchlorid oder Kaliumchlorid, oder aber durch Eindampfen der Reaktionslösung selbst, beispielsweise durch Sprühtrocknung. Falls die letztgenannte Art der Isolierung gewählt wird, empfiehlt es sich vielfach, vor dem Eindampfen eventuell in den Lösungen vorhandene Sulfatmengen durch Fällung als Calciumsulfat und Abtrennung durch Filtration zu entfernen.

Gegenstand der vorliegenden Erfindung ist deshalb auch die Verwendung der Verbindungen (1) zum Färben (einschließlich Bedrucken) von hydroxy- und/oder carbonamidgruppenhaltigen Materialien bzw. Verfahren zu deren Anwendung auf diesen Substraten. Bevorzugt kommen die Materialien in Form von Fasermateialien zur Anwendung, insbesondere in Form von Textilfasern, wie Garnen, Wickelkörpern und Geweben. Hierbei kann man analog bekannten Verfahrensweisen vorgehen.

Hydroxygruppenhaltige Materialien sind solche natürlichen oder synthetischen Ursprungs, wie beispielsweise Cellulosefasermaterialien oder deren Regeneratprodukte und Polyvinylalkohole. Cellulosefasermaterialien sind vorzugsweise Baumwolle, aber auch andere Pflanzenfasern, wie Leinen, Hanf, Jute und

Ramiefasern; regenerierte Cellulosefasern sind beispielsweise Zellwolle und Viskosekunstseide.

Carbonamidgruppenhaltige Materialien sind beispielsweise synthetische und natürliche Polyamide und Polyurethane, insbesondere in Form von Fasern, beispielsweise Wolle und andere Tierhaare, Seide, Leder, Polyamid-6,6, Polyamid-6, Polyamid-11 und Polyamid-4.

Die Verbindungen (1) lassen sich, gemäß der erfindungsgemäßen Anwendung, auf den genannten Substraten, insbesondere auf den genannten Fasermaterialien, nach den für wasserlösliche, faserreaktive Farbstoffe bekannten Anwendungstechniken applizieren und fixieren, so beispielsweise, indem man die Verbindung (1) in gelöster Form auf das Substrat aufbringt oder sie darin einbringt und sie auf diesem oder in diesem, gegebenenfalls durch Hitzeeinwirkung und/oder gegebenenfalls durch Einwirkung eines alkalisch wirkenden Mittels, fixiert. Solche Färbe- und Fixierweisen sind in der Literatur zahlreich beschrieben (s. bspw. europäische Patentanmeldungs-Veröffentlichung Nr. 0 181 505 A2).

Die erfindungsgemäßen Färbungen besitzen, insbesondere auf Cellulosefasermaterialien, gute Lichtechtheiten sowohl im trockenen Zustand der Färbung als auch im nassen, beispielsweise mit einer Schweißlösung befeuchteten, Zustand sowie gute Naßechtheiten, wie beispielsweise gute Waschechtheiten bei 60 bis 95°C, auch in Gegenwart von Perboraten, gute saure und alkalische Walk-, Überfärbe- und Schweißechtheiten, eine hohe Dampfbeständigkeit, gute Alkali-, Säure-, Wasser- und Seewasserechtheiten, des weiteren eine gute Plissierechtheit, Bügelechtheit und Reibechtheit. Ebenso besitzen sie eine gute Säurelagerbeständigkeit ("acid fading") beim Lagern von feuchten, noch Essigsäure enthaltendem, gefärbtem Material.

Die nachstehenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wie Kilogramm zu Liter.

Die in diesen Beispielen formelmäßig beschriebenen Verbindungen sind in Form der freien Säuren angegeben; im allgemeinen werden sie in Form ihrer Alkalimetallsalze, wie Lithium-, Natrium- oder Kaliumsalze, hergestellt und isoliert und in Form ihrer Salze zum Färben verwendet. Ebenso können die in den nachfolgenden Beispielen, insbesondere Tabellenbeispielen, in Form der freien Säure genannten Ausgangsverbindungen und Komponenten als solche oder in Form ihrer Salze, vorzugsweise Alkalimetallsalze, in die Synthese eingesetzt werden.

Die für die erfindungsgemäßen Verbindungen angegebenen Absorptionsmaxima ($\lambda_{max}$) im sichtbaren Bereich wurden anhand derer Alkalimetallsalze in wäßriger Lösung ermittelt. In den Tabellenbeispielen sind die $\lambda_{max}$-Werte bei der Farbtonangabe in Klammern gesetzt; die Wellenlängenangabe bezieht sich auf nm.

**Beispiel A**

Eine Lösung mit einem pH-Wert von 9 von 280 Teilen β-[4-(β'-Hydroxyethylsulfonyl)-phenyl]-ethylamin-Hydrochlorid in etwa 1 500 Teilen Wasser werden langsam bei 5 bis 10°C unter Einhaltung eines pH-Wertes mittels etwa 3 000 Teilen einer 5 %igen wäßrigen Natronlauge unter guten Rühren mit einer Lösung von 237 Teilen 4-Nitrophenoxyessigsäurechlorid in 500 Volumenteilen Toluol versetzt. Man rührt den Ansatz anschließend noch etwa 4 Stunden bei 5 bis 10°C nach, saugt vom ausgefallenen Produkt ab und kristallisiert es aus Methanol um.

Es besitzt die Formel

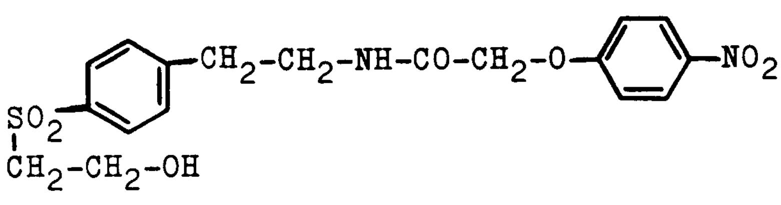

und hat folgende physikalische Daten:

Schmelzpunkt: 113°C ;

Banden im IR-Spektrum:

3352 $cm^{-1}$ (NH) ; 2928 $cm^{-1}$ (CH) ; 1655 $cm^{-1}$ (CO) ;

| Elementaranalyse ($C_{18}H_{20}N_2O_7S$): | | | | |
|---|---|---|---|---|
| ber.: | C 52,9 % | H 4,9 % | N 6,8 % | S 7,8 % |
| gef.: | C 52,7 % | H 4,9 % | N 6,7 % | S 7,6 % . |

**Beispiel B**

56 Teile der Nitroverbindung von Beispiel A werden in 300 Teilen Methanol suspendiert und mittels Wasserstoff über Raney-Nickel bei einer Temperatur von 60 °C und einem Wasserstoffdruck von 60 bar reduziert. Nach Reaktionsende wird der Katalysator bei 90 °C durch Filtration entfernt und das Filtrat auf 20 °C abkühlen lassen. Das auskristallisierte Produkt wird abgesaugt und mit 100 Teilen Methanol nachgewaschen. Es besitzt die Formel

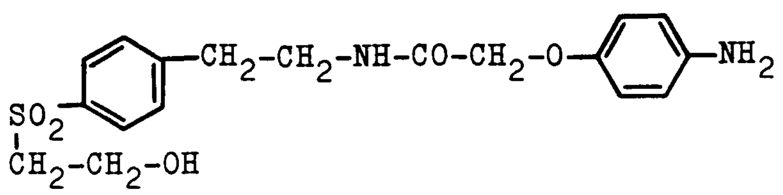

und hat folgende physikalische Daten:
Schmelzpunkt: 136 °C ;
Banden im IR-Spektrum:
3376 $cm^{-1}$ (NH) ; 3300 $cm^{-1}$ (NH) ; 2960 $cm^{-1}$ (CH); 1655 $cm^{-1}$ (CO) .

**Beispiel C**

37 Teile der Anilinverbindung von Beispiel B werden in 148 Teile Schwefelsäure-Monohydrat bei 10 °C eingetragen. Man erwärmt die erhaltene Suspension anschließend langsam auf 20 °C und rührt sie noch drei Stunden weiter, gibt sie danach auf 1 600 Teile Eis und saugt den Niederschlag ab. Die Verbindung besitzt die Formel

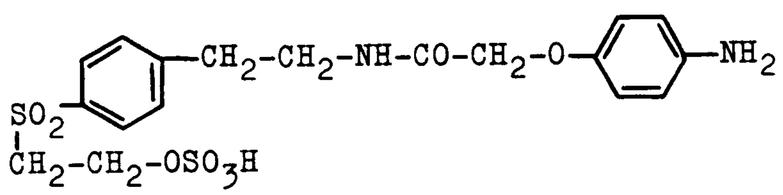

und zeigt folgende Banden im IR-Spektrum:
3390 $cm^{-1}$ (NH) ; 2944 $cm^{-1}$ (CH) ; 1660 $cm^{-1}$ (CO).

**Beispiel 1**

Eine wäßrige Lösung mit einem pH-Wert von 6,8 von 64,8 Teilen der Anilinverbindung von Beispiel C in 3 200 Teilen Wasser wird zunächst mit 20 Teilen einer wäßrigen 5n-Natriumnitritlösung und anschließend bei 5 °C mit 70 Teilen einer wäßrigen konzentrierten Salzsäure versetzt. Nach beendeter Diazotierungsreaktion entfernt man überschüssiges Nitrit mit Amidosulfonsäure und gibt zu der Diazoniumsalz-Suspension bei 5 °C und unter Einhaltung eines pH-Wertes von 6 51 Teile 1-Acetylamino-8-naphthol-3,6-disulfonsäure hinzu und rührt den Ansatz anschließend noch etwa 24 Stunden bei 15 bis 20 °C nach, stellt den pH-Wert auf 4,5 bis 5 und isoliert die erfindungsgemäße Azoverbindung durch Aussalzen mit Natriumchlorid und Filtration.

Sie hat, in Form der freien Säure geschrieben, die Formel

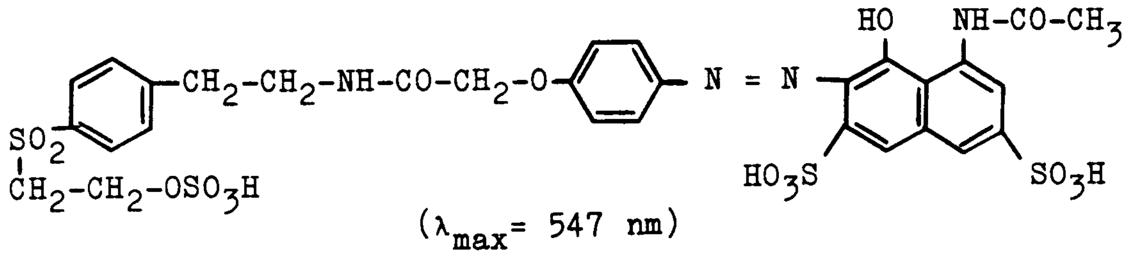

($\lambda_{max}$= 547 nm)

und besitzt sehr gute faserreaktive Farbstoffeigenschaften. Bei Anwendung der für faserreaktive Farbstoffe bekannten Applikations- und Fixierverfahren erhält man mit der erfindungsgemäßen Azoverbindung auf den in der Beschreibung genannten Materialien, wie insbesondere Cellulosefasermaterialien, beispielsweise Baumwolle, farbstarke, blaustichig rote Färbungen und Drucke mit guten Echtheitseigenschaften.

**Beispiele 2 bis 52**

In den nachfolgenden Tabellenbeispielen sind weitere erfindungsgemäße Azoverbindungen entsprechend der in Form der freien Säure geschriebenen allgemeinen Formel (A)

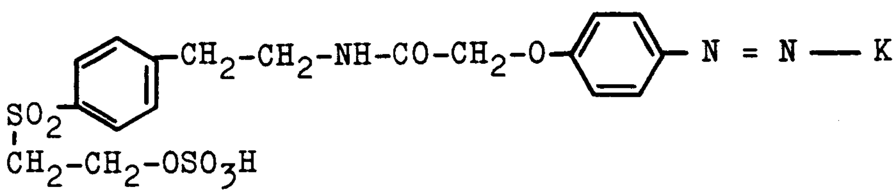

(A)

mit Hilfe ihrer Kupplungskomponente H-K beschrieben. Sie lassen sich in erfindungsgemäßer Weise, beispielsweise analog dem obigen Beispiel 1, herstellen und besitzen ebenfalls sehr gute faserreaktive Farbstoffeigenschaften.

Sie liefern farbstarke Färbungen und Drucke auf den in der Beschreibung genannten Materialien, wie insbesondere Cellulosefasermaterialien, nach den für faserreaktive Farbstoffe üblichen Applikations- und Fixiermethoden mit den in dem jeweiligen Tabellenbeispiel angegebenen Farbton (hier auf Baumwolle) mit guten Echtheitseigenschaften; die in Klammern angegebenen Werte sind die Absorptionsmaxima ($\lambda_{max}$) in nm.

| Bsp. | Kupplungskomponente H-K in Formel (A) | Farbton |
|---|---|---|
| 2 | 6-Sulfo-3-benzoylamino-8-naphthol | rot |
| 3 | 1-(4'-β-Sulfatoethylsulfonyl-phenyl)-3-methyl-5-pyrazolon | gelb (419) |
| 4 | 4,6-Disulfo-1-acetylamino-8-naphthol | blaustichig rot (547) |
| 5 | 3,6-Disulfo-2-amino-naphthalin | rot |
| 6 | 5,7-Disulfo-2-amino-naphthalin | marineblau |
| 7 | 4-Sulfo-1-naphthol | rot |
| 8 | 3,6-Disulfo-1-naphthol | rot |
| 9 | 1-(N-ß-Sulfoethyl)-4-methyl-2-hydroxy-6-pyridon | orange |
| 10 | 3,6,8-Trisulfo-2-amino-naphthalin | rot |
| 11 | 6-Sulfo-3-acetylamino-8-naphthol | rot (499) |
| 12 | N,N-Bis-(ß-sulfatoethyl)-anilin | orange |
| 13 | N-Ethyl-N-(ß-sulfatoethyl)-anilin | rot |
| 14 | 6-Sulfo-2-acetylamino-8-naphthol | rot (509) |
| 15 | 3,6-Disulfo-1-benzoylamino-8-naphthol | blaustichig rot (551) |
| 16 | 4,6-Disulfo-1-benzoylamino-8-naphthol | blaust. rot |
| 17 | N,N-Bis-(ß-sulfatoethyl)-3-chlor-anilin | orange |
| 18 | 5-Sulfo-1-naphthol | rot |
| 19 | 3,6-Disulfo-1-amino-2-{2'-sulfo-5'-[5"-(ß-sulfatoethylsulfonyl)-1",2",3"-benzotriazol-1"-yl]-phenyl-diazoyl}-8-naphthol | grünstichig blau |

| Bsp. | Kupplungskomponente H-K in Formel (A) | Farbton |
|---|---|---|
| 20 | 3,6-Disulfo-1-amino-2-[4'-(ß-sulfato-ethylsulfonyl)-phenyl-diazoyl]-8-naphthol | grünstichig blau (627) |
| 21 | 6-Sulfo-2-methylamino-8-naphthol | rot |
| 22 | 4-Sulfo-diphenylamin | rot |
| 23 | 5-Sulfo-2-acetylamino-7-naphthol | rot |
| 24 | 3,6-Disulfo-2-acetylamino-8-naphthol | rot |
| 25 | 2,4-Disulfo-1-amino-8-naphthol | blau |
| 26 | 3,6-Disulfo-1-amino-8-naphthol (in 7-Stellung gekuppelt) | blau |
| 27 | 4,6-Disulfo-1-amino-8-naphthol (in 7-Stellung gekuppelt) | blau |
| 28 | 3,6-Disulfo-1-phenylureido-8-naphthol | blau |
| 29 | 3-Sulfo-1-naphthol | rot |
| 30 | 5-Sulfo-2-naphthol | rot |
| 31 | 6-Sulfo-2-naphthol | blaust. rot |
| 32 | 8-Sulfo-2-naphthol | blaust. rot |
| 33 | 3,6-Disulfo-1-acetylamino-8-naphthol | blau |
| 34 | N,N-Bis-(ß-hydroxyethyl)-anilin | rot |
| 35 | 3,6,8-Trisulfo-1-naphthol | blaust. rot |
| 36 | 3,6-Disulfo-2-naphthol | rot |
| 37 | 3,6-Disulfo-1-acetylamino-8-naphthol | blau |
| 38 | 4,6-Disulfo-1-acetylamino-8-naphthol | blau |
| 39 | N-Ethyl-N-(3'-sulfobenzyl)-anilin | rot |
| 40 | 5-Sulfo-1,4-dimethyl-2-hydroxy-6-pyridon | gelb |
| 41 | 2-Carboxy-acetoacetylanilin | gelb |
| 42 | 5-Sulfo-8-hydroxy-chinolin | rot |
| 43 | 3-Sulfo-acetoacetylanilin | gelb |
| 44 | 1-(4'-Sulfophenyl)-3-methyl-5-pyrazolon | gelb (416) |
| 45 | 1-(2'-Chlor-5'-sulfophenyl)-3-methyl-5-pyrazolon | gelb |
| 46 | 1-(2',5'-Disulfophenyl)-3-methyl-5-pyrazolon | gelb |
| 47 | 1-(4',8'-Disulfo-naphth-2'-yl)-3-methyl-5-pyrazolon | gelb |

| Bsp. | Kupplungskomponente H-K in Formel (A) | Farbton |
|---|---|---|
| 48 | 1-(4'-Sulfophenyl)-3-methyl-5-amino-pyrazol | gelb |
| 49 | 3,6-Disulfo-1-[4'-chlor-6'-(3"-sulfo-phenyl)-amino-1',3',5'-triazin-2'-yl]-amino-8-naphthol | blaustichig rot |
| 50 | 3,6-Disulfo-1-{4'-chlor-6'-[4"-(ß-sulfatoethylsulfonyl)-phenyl]-amino-1',3',5'-triazin-2'-yl}-amino-8-naphthol | blaustichig rot (555) |
| 51 | 6,8-Disulfo-2-naphthol | rot |
| 52 | 6-Sulfo-2-benzoylamino-8-naphthol | rot |

## Patentansprüche

1. Eine wasserlösliche Azoverbindung, die der allgemeinen Formel (1)

   D — N = N — K (1)

   entspricht, in welcher bedeuten:

   D ist ein Rest der allgemeinen Formel (2)

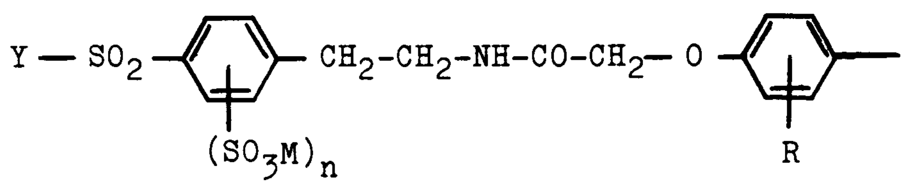

(2)

in welcher

Y die Vinylgruppe oder eine Gruppe der allgemeinen Formel (3)

$-CH_2-CH_2-X$ (3)

ist, in welcher

X ein Substituent ist, der durch ein Alkali unter Bildung der Vinylgruppe eliminierbar ist,

R ein Wasserstoffatom, eine Nitrogruppe, eine Alkylgruppe von 1 bis 4 C-Atomen, eine Alkoxygruppe von 1 bis 4 C-Atomen, eine Carboxygruppe, eine Hydroxygruppe oder ein Halogenatom,

n für die Zahl 0 oder 1 steht und

M ein Wasserstoffatom oder ein salzbildendes Metallatom ist;

K ist ein Rest einer einfach ankuppelbaren wasserlöslichen Kupplungskomponente, die noch eine Azogruppe enthalten kann, oder der Rest einer doppelankuppelbaren wasserlöslichen Kupplungskomponente, jeweils aus der Reihe der Aminobenzole, der Phenole, insbesondere deren Sulfonsäuren und Carbonsäuren, der Naphthole, insbesondere deren Sulfonsäuren und Carbonsäuren, der Aminonaphthole, insbesondere deren Sulfonsäuren, der Acylamino-naphthole, insbesondere deren Sulfonsäuren, mit dem Acylrest einer Alkan- oder Alkencarbonsäure

mit jeweils 1 bis 4 bzw. 2 bis 4 C-Atomen im Alkyl-bzw. Alkenylrest oder einer aromatischen Carbonsäure oder einer aromatischen Sulfonsäure oder einer N-substituierten Carbaminsäure oder aus der Reihe der Dihydroxynaphthalinsulfonsäuren, der Phenylazo- und Naphthylazo-aminonaphtholsulfonsäuren, der 5-Pyrazolone und 5-Aminopyrazole, der Acetoacetylarylide, der 2-Hydroxy-6-pyridone und der Hydroxychinoline, wobei K neben den in Farbstoffen üblichen Substituenten auch eine oder mehrere faserreaktive Gruppen enthalten kann.

**2.** Verbindung nach Anspruch 1, dadurch gekennzeichnet, daß K ein Rest der allgemeinen Formel

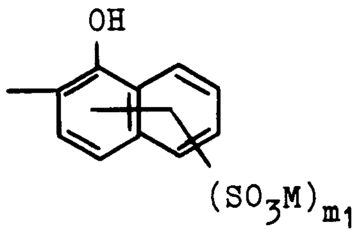

(4f')

ist, in welcher $m_1$ für die Zahl 1, 2 oder 3 steht.

**3.** Verbindung nach Anspruch 1, dadurch gekennzeichnet, daß K ein Rest der allgemeinen Formel

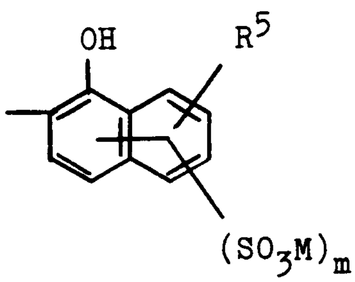

(4h)

ist, in welcher $R^5$ die Benzoylaminogruppe oder eine Alkanoylaminogruppe von 2 bis 5 C-Atomen ist und m für die Zahl 1 oder 2 steht.

**4.** Verbindung nach Anspruch 1, dadurch gekennzeichnet, daß K ein Rest der allgemeinen Formel

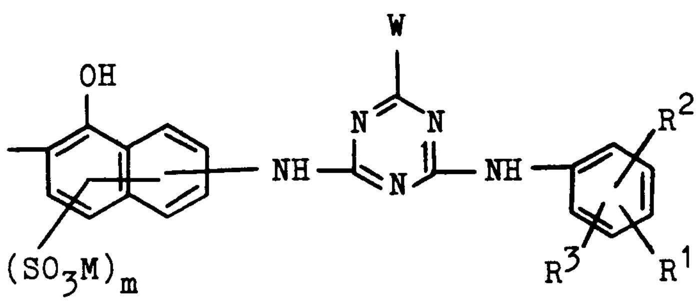

(4v)

ist, in welcher m für die Zahl 1 oder 2 steht, M die in Anspruch 1 genannte Bedeutung besitzt, W eine Sulfogruppe, eine Alkylsulfonylgruppe von 1 bis 4 C-Atomen, die Phenylsulfonylgruppe oder ein Brom- oder Fluor- oder Chloratom ist, $R^1$ die Gruppe $-SO_2-Y$ mit Y einer der in Anspruch 1 genannten Bedeutungen ist, $R^2$ ein Wasserstoffatom, eine Alkylgruppe von 1 bis 4 C-Atomen, eine Alkoxygruppe von 1 bis 4 C-Atomen, ein Chlor- oder Bromatom oder eine Carboxy-, Sulfo-oder Nitrogruppe ist und $R^3$ ein Wasserstoffatom bedeutet.

**5.** Verbindung nach Anspruch 1, dadurch gekennzeichnet, daß K ein Rest der allgemeinen Formel

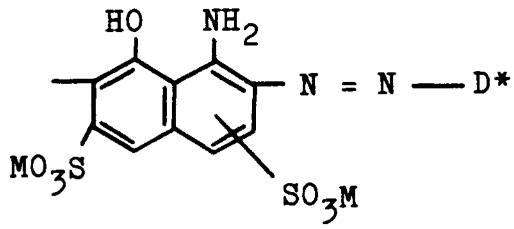

(4p')

ist, in welcher M eine der in Anspruch 1 genannten Bedeutungen besitzt und D* ein Phenylrest ist, der durch 1, 2 oder 3 Substituenten aus der Gruppe Alkyl von 1 bis 4 C-Atomen, Alkoxy von 1 bis 4 C-Atomen, Chlor, Brom, Hydroxy, Carboxy, Sulfo, Carbamoyl, Sulfamoyl und Alkanoylamino von 2 bis 5 C-Atomen und/oder durch eine Gruppe der allgemeinen Formel $-SO_2-Y$ mit Y einer in Anspruch 1 genannten Bedeutung substituiert sein kann, oder ein Naphthylrest ist, der durch 1, 2 oder 3 Sulfogruppen oder durch 1 oder 2 Sulfogruppen und 1 oder 2 Gruppen der allgemeinen Formel $-SO_2-Y$ mit Y einer in Anspruch 1 genannten Bedeutung oder nur durch eine solche Gruppe $-SO_2-Y$ substituiert ist.

**6.** Verbindung nach Anspruch 4, dadurch gekennzeichnet, daß W ein Fluor- oder Chloratom ist.

**7.** Verbindung nach Anspruch 5, dadurch gekennzeichnet, daß D* ein Phenylrest ist, der in para-Stellung zur Azogruppe durch eine Gruppe der allgemeinen Formel $-SO_2-Y$ mit Y der in Anspruch 1 genannten Bedeutung substituiert ist.

**8.** Verbindung nach mindestens einem der Ansprüche 1 bis 7, dadurch gekennzeichnet, daß n für die Zahl Null steht.

**9.** Verbindung nach mindestens einem der Ansprüche 1 bis 8, dadurch gekennzeichnet, daß R ein Wasserstoffatom bedeutet.

**10.** Verbindung nach mindestens einem der Ansprüche 1 bis 9, dadurch gekennzeichnet, daß Y für die Vinylgruppe oder eine β-Sulfatoethyl-Gruppe steht.

**11.** Verbindung nach mindestens einem der Ansprüche 1 bis 10, dadurch gekennzeichnet, daß Y eine β-Sulfatoethyl-Gruppe ist.

**12.** Verbindung nach mindestens einem der Ansprüche 1 bis 11, dadurch gekennzeichnet, daß M für ein Wasserstoffatom oder ein Alkalimetall steht.

**13.** Verfahren zur Herstellung einer Verbindung der in Anspruch 1 genannten und definierten allgemeinen Formel (1), dadurch gekennzeichnet, daß man eine Diazoniumverbindung einer Aminoverbindung der allgemeinen Formel (5)

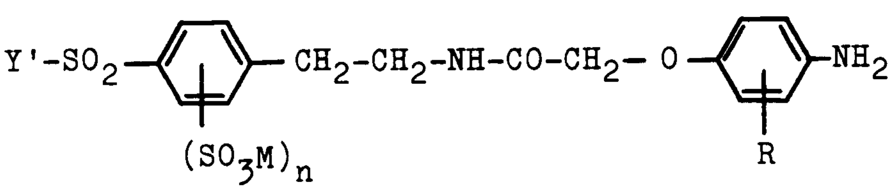

(5)

in welcher Y' eine der Bedeutungen von Y hat oder die β-Sydroxyethyl-Gruppe ist und R, M und n die in Anspruch 1 genannten Bedeutungen haben, mit einer Kupplungskomponente der allgemeinen Formel H-K mit K der in Anspruch 1 genannten Bedeutung kuppelt und gegebenenfalls die gebildete Azoverbindung mit einer weiteren Diazoverbindung oder Kupplungskomponente umsetzt und im Falle,

daß Y' die β-Hydroxyethyl-Gruppe ist, diese in der gebildeten Azoverbindung in eine Gruppe Y der in Anspruch 1 genannten Bedeutung überführt.

**14.** Verwendung einer Verbindung entsprechend der allgemeinen Formel (1) von Anspruch 1 zum Färben (einschließlich Bedrucken) von hydroxy- und/oder carbonamidgruppenhaltigem Material, insbesondere Fasermaterial.

**15.** Verfahren zum Färben (einschließlich Bedrucken) von hydroxy- und/oder carbonamidgruppenhaltigem Material, insbesondere Fasermaterial, bei welchem man einen Farbstoff auf das Material aufbringt oder darin einbringt und ihn mittels Wärme und/oder mit Hilfe eines alkalisch wirkenden Mittels fixiert, dadurch gekennzeichnet, daß man als Farbstoff eine Verbindung entsprechend der allgemeinen Formel (1) von Anspruch 1 einsetzt.

**16.** Eine Verbindung, die der allgemeinen Formel

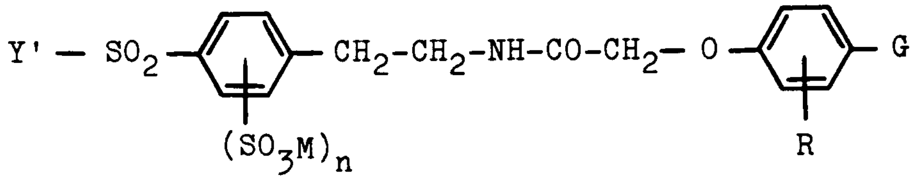 (5')

entspricht, in welcher

G die Aminogruppe oder die Nitrogruppe ist,
R ein Wasserstoffatom, eine Nitrogruppe, eine Alkylgruppe von 1 bis 4 C-Atomen, eine Alkoxygruppe von 1 bis 4 C-Atomen, eine Carboxygruppe, eine Hydroxygruppe oder ein Halogenatom ist,
M ein Wasserstoffatom oder ein salzbildendes Metallatom, vorzugsweise ein Alkalimetallatom, ist,
n für die Zahl Null oder 1 steht und
Y' die β-Hydroxyethyl-Gruppe oder die Vinylgruppe oder eine Ethylgruppe ist, die in β-Stellung einen Substituenten enthält, der durch ein Alkali unter Bildung der Vinylgruppe eliminierbar ist.

**17.** Eine Verbindung nach Anspruch 16, dadurch gekennzeichnet, daß Y' die β-Hydroxyethyl- oder die β-Sulfatoethyl-Gruppe oder die Vinylgruppe bedeutet.

**18.** Eine Verbindung nach Anspruch 16 oder 17, dadurch gekennzeichnet, daß n für die Zahl Null steht.

**19.** Eine Verbindung nach mindestens einem der Ansprüche 16 bis 18, dadurch gekennzeichnet, daß R ein Wasserstoffatom ist.

**20.** Verfahren zur Herstellung einer Verbindung von Anspruch 16, dadurch gekennzeichnet, daß man eine Verbindung der allgemeinen Formel (6)

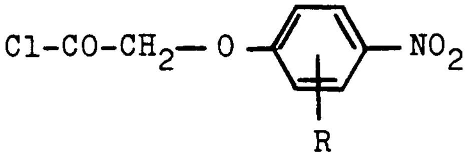 (6)

in welcher R die in Anspruch 1 genannte Bedeutung hat, mit einer Aminoverbindung der allgemeinen Formel

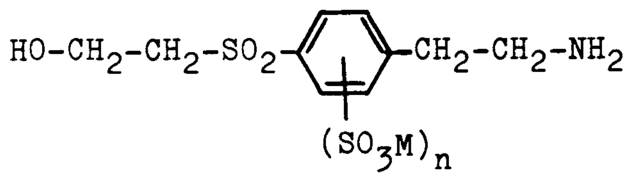

(7)

in welcher M und n die in Anspruch 1 genannten Bedeutungen haben, umsetzt und in der so erhaltenen Verbindung der allgemeinen Formel (8)

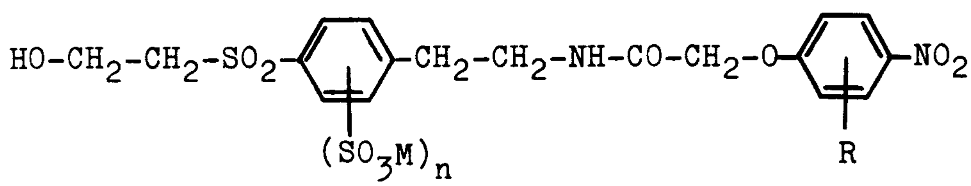

(8)

in welcher R, M und n die in Anspruch 16 genannten Bedeutungen haben, die Nitrogruppe zur Verbindung der allgemeinen Formel (5A)

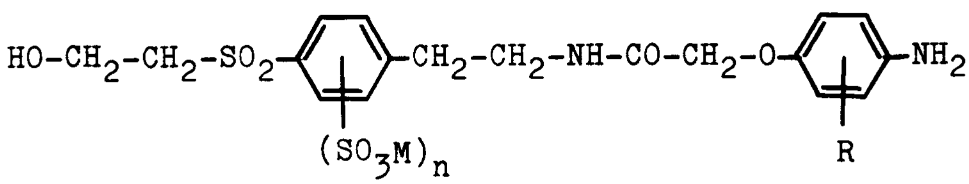

(5A)

mit R, M und n der obengenannten Bedeutung reduziert und gegebenenfalls in den Verbindungen der Formel (8) und (5A) die β-Hydroxyethylsulfonyl-Gruppe in eine Gruppe der Formel Y-$SO_2$- mit Y eine der in Anspruch 16 genannten Bedeutungen überführt.

**21.** Verwendung einer Verbindung von Anspruch 16 zur Synthese von Farbstoffen, insbesondere von Azofarbstoffen.

**Claims**

**1.** A water-soluble azo compound which corresponds to the formula (1)

D—N = N—K (1)

in which:
D is a radical of the formula (2)

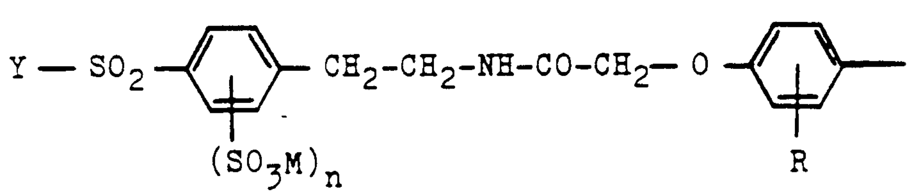

(2)

in which
Y is the vinyl group or a group of the formula (3)

$-CH_2-CH_2-X$ (3)

in which
X is a substituent which can be removed by an alkali to form the vinyl group,
R is a hydrogen atom, a nitro group, an alkyl group having 1 to 4 carbon atoms, an alkoxy group having 1 to 4 carbon atoms, a carboxyl group, a hydroxyl group or a halogen atom,
n represents the number 0 or 1, and
M is a hydrogen atom or a salt-forming metal atom;
K is a radical of a water-soluble coupling component which can be coupled in one position and can also contain an azo group, or the radical of a water-soluble coupling component which can be coupled in two positions, in each case from the series comprising aminobenzenes, phenols, in particular sulfonic acids and carboxylic acids thereof, naphthols, in particular sulfonic acids and carboxylic acids thereof, aminonaphthols, in particular sulfonic acids thereof, and acylamino-naphthols, in particular sulfonic acids thereof, with the acyl radical of an alkane- or alkenecarboxylic acid having in each case 1 to 4 or 2 to 4 carbon atoms in the alkyl or alkenyl radical respectively, or of an aromatic carboxylic acid or of an aromatic sulfonic acid, or of an N-substituted carbamic acid, or from the series comprising dihydroxynaphthalenesulfonic acids, phenylazo- and naphthylazo-aminonaphtholsulfonic acids, 5-pyrazolones and 5-aminopyrazoles, acetoacetyl arylides, 2-hydroxy-6-pyridones and hydroxyquinolines, it also being possible for K to contain one or more fiber-reactive groups, in addition to the substituents customary in dyestuffs.

**2.** A compound as claimed in claim 1, wherein K is a radical of the formula

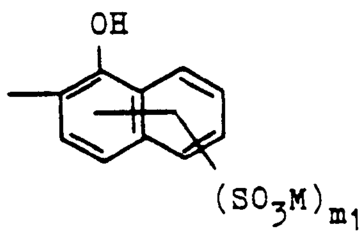

(4f')

in which $m_1$ represents the number 1, 2 or 3.

**3.** A compound as claimed in claim 1, wherein K is a radical of the formula

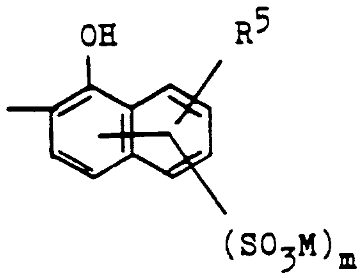

(4h)

in which $R^5$ is the benzoylamino group or an alkanoylamino group having 2 to 5 carbon atoms and m represents the number 1 or 2.

**4.** A compound as claimed in claim 1, wherein K is a radical of the formula

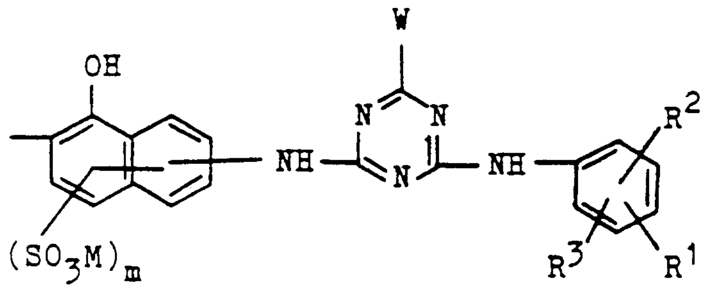

(4v)

in which m represents the number 1 or 2, M has the meaning given in claim 1, W is a sulfo group, an alkylsulfonyl group having 1 to 4 carbon atoms, the phenylsulfonyl group or a bromine or fluorine or chlorine atom, $R^1$ is the group $-SO_2-Y$, where Y has one of the meanings given in claim 1, $R^2$ is a hydrogen atom, an alkyl group having 1 to 4 carbon atoms, an alkoxy group having 1 to 4 carbon atoms, a chlorine or bromine atom or a carboxyl, sulfo or nitro group and $R^3$ is a hydrogen atom.

**5.** A compound as claimed in claim 1, in which K is a radical of the formula

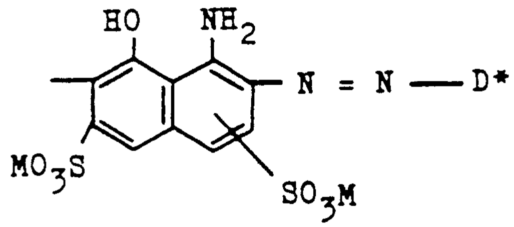

(4p')

in which M has one of the meanings mentioned in claim 1 and D* is a phenyl radical, which can be substituted by 1, 2 or 3 substituents from the group comprising alkyl having 1 to 4 carbon atoms, alkoxy having 1 to 4 carbon atoms, chlorine, bromine, hydroxyl, carboxyl, sulfo, carbamoyl, sulfamoyl and alkanoylamino having 2 to 5 carbon atoms and/or by a group of the formula $-SO_2-Y$, where Y has a meaning given in claim 1, or is a naphthyl radical, which is substituted by 1, 2 or 3 sulfo groups or by 1 or 2 sulfo groups and 1 or 2 groups of the formula $-SO_2-Y$, where Y has a meaning given in claim 1, or only by one such group $-SO_2-Y$.

**6.** A compound as claimed in claim 4, wherein W is a fluorine or chlorine atom.

**7.** A compound as claimed in claim 5, wherein D* is a phenyl radical, which is substituted in the para-position relative to the azo group by a group of the formula $-SO_2-Y$, where Y has the meaning given in claim 1.

**8.** A compound as claimed in any one of claims 1 to 7, wherein n represents the number zero.

**9.** A compound as claimed in any one of claims 1 to 8, wherein R is a hydrogen atom.

**10.** A compound as claimed in any one of claims 1 to 9, wherein Y is the vinyl group or a $\beta$-sulfatoethyl group.

**11.** A compound as claimed in any one of claims 1 to 10, wherein Y is a $\beta$-sulfatoethyl group.

**12.** A compound as claimed in any one of claims 1 to 11, wherein M is a hydrogen atom or an alkali metal.

**13.** A process for the preparation of a compound of the formula (1) mentioned and defined in claim 1, which comprises coupling a diazonium compound of an amino compound of the formula (5)

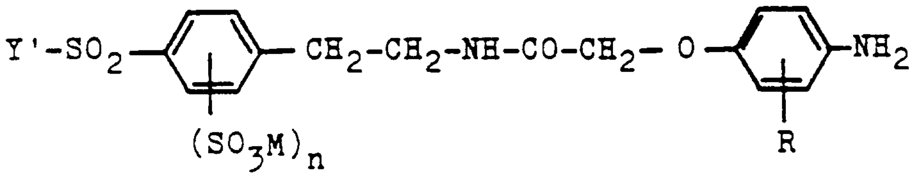
(5)

in which Y' has one of the meanings of Y or is the $\beta$-hydroxyethyl group and R, M and n have the meanings given in claim 1, with a coupling component of the formula H-K, where K has the meaning given in claim 1, and if appropriate reacting the azo compound formed with another diazo compound or coupling component and, if Y' is the $\beta$-hydroxyethyl group, converting this in the azo compound formed into a group Y of the meaning given in claim 1.

**14.** The use of a compound corresponding to the formula (1) of claim 1 for dyeing (including printing) material containing hydroxyl and/or carboxamide groups, in particular fiber material.

**15.** A process for dyeing (including printing) material containing hydroxyl and/or carboxamide groups, in particular fiber material, in which a dyestuff is applied to the material or incorporated therein and is fixed by means of heat and/or with the aid of an alkaline agent, which comprises using a compound corresponding to the formula (1) of claim 1 as the dyestuff.

**16.** A compound which corresponds to the formula

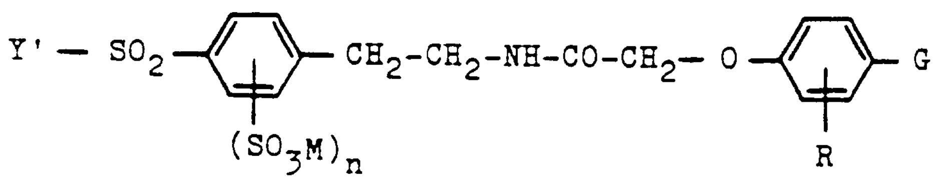
(5')

in which
G is the amino group or the nitro group,
R is a hydrogen atom, a nitro group, an alkyl group having 1 to 4 carbon atoms, an alkoxy group having 1 to 4 carbon atoms, a carboxyl group, a hydroxyl group or a halogen atom,
M is a hydrogen atom or a salt-forming metal atom, preferably an alkali metal atom,
n represents the number zero or 1 and
Y' is the $\beta$-hydroxyethyl group or the vinyl group or an ethyl group which contains a substituent in the $\beta$-position which can be removed by an alkali to form the vinyl group.

**17.** A compound as claimed in claim 16, wherein Y' is the $\beta$-hydroxyethyl or the $\beta$-sulfatoethyl group or the vinyl group.

**18.** A compound as claimed in either of claims 16 and 17, wherein n represents the number zero.

**19.** A compound as claimed in any one of claims 16 to 18, wherein R is a hydrogen atom.

**20.** A process for the preparation of a compound of claim 16, which comprises reacting a compound of the formula (6)

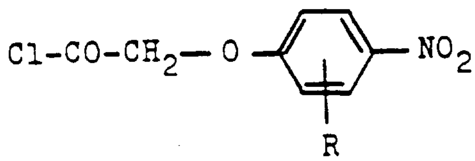

(6)

in which R has the meaning given in claim 1, with an amino compound of the formula

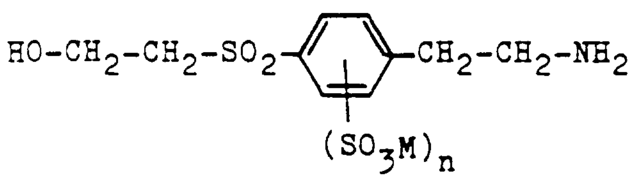

(7)

in which M and n have the meanings given in claim 1, and reducing the nitro group in the resulting compound of the formula (8)

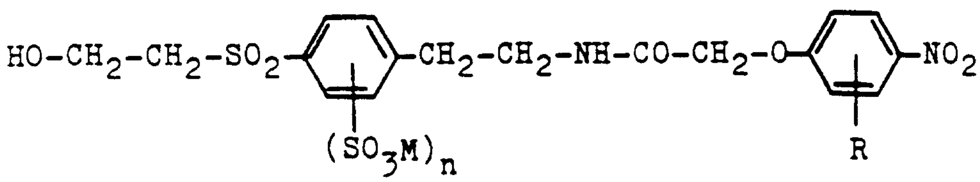

(8)

in which R, M and n have the meanings given in claim 16, to give the compound of the formula (5A)

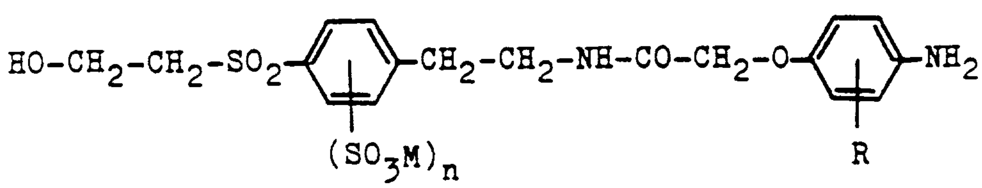

(5A)

where R, M and n have the abovementioned meaning, and if appropriate converting the $\beta$-hydroxyethylsulfonyl group in the compounds of the formula (8) and (5A) into a group of the formula Y-$SO_2$-, where Y has one of the meanings given in claim 16.

**21.** The use of a compound as claimed in claim 16 for the synthesis of dyestuffs, in particular azo dyestuffs.

**Revendications**

**1.** Composé azoïque soluble dans l'eau correspondant à la formule générale (1)

D - N = N - K (1)

dans laquelle
D est un reste de formule générale (2)

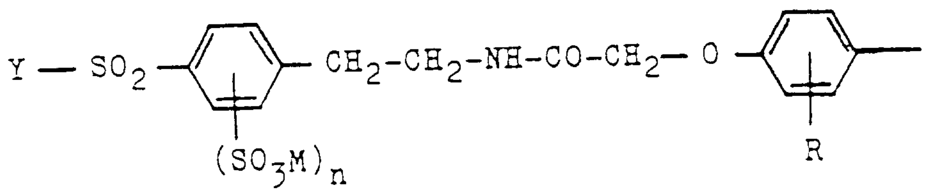

(2)

dans laquelle

Y représente le groupe vinyle ou un groupe de formule générale (3)

$$- CH_2 - CH_2 X \quad (3)$$

dans laquelle

X est un substituant qui peut être éliminé par une base avec formation d'un groupe vinyle,

R représente un atome d'hydrogène, un groupe nitro, un groupe alkyle de 1 à 4 atomes de carbone, un groupe alcoxy de 1 à 4 atomes de carbone, un groupe carboxy, un groupe hydroxy ou un atome d'halogène,

n est le nombre 0 ou 1 et

M représente un atome d'hydrogène ou un atome métallique formant un sel;

K est un reste d'un constituant de copulation soluble dans l'eau pouvant subir une seule copulation et qui peut contenir un autre groupe azoïque, ou le reste d'un constituant de copulation soluble dans l'eau pouvant subir deux copulations, dans chaque cas de la série des aminobenzènes, des phénols, en particulier de leurs acides sulfoniques et de leurs acides carboxyliques, des naphtols, en particulier de leurs acides sulfoniques et de leurs acides carboxyliques, des aminonaphtols, en particulier de leurs acides sulfoniques, des acylaminonaphtols, en particulier de leurs acides sulfoniques, avec le reste acyle d'un acide alcane- ou alcènecarboxylique ayant chacun respectivement 1 à 4 atomes de carbone ou 2 à 4 atomes de carbone dans le reste alkyle ou alcényle ou d'un acide carboxylique aromatique ou d'un acide sulfonique aromatique ou d'un acide carbamique N-substitué, ou de la série des acides dihydroxynaphtalènesulfoniques, des acides phénylazo- et naphtylazoaminonaphtolsulfoniques, des 5-pyrazolones et des 5-aminopyrazoles, des acétoacétylarylides, des 2-hydroxy-6-pyridones et des hydroxyquinoléines, K pouvant contenir, en plus des substituants classiques des colorants, un ou plusieurs groupes réactifs avec les fibres.

**2.** Composé selon la revendication 1, caractérisé en ce que K est un reste de formule générale

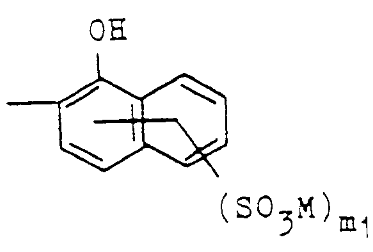

(4f)

dans laquelle $m_1$ représente le nombre 1, 2 ou 3.

**3.** Composé selon la revendication 1, caractérisé en ce que K est un reste de formule générale

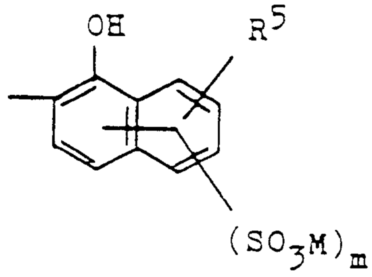

(4h)

dans laquelle $R^5$ représente le groupe benzoylamino ou un groupe alcanoyalmino de 2 à 5 atomes de carbone et m représente le nombre 1 ou 2.

**4.** Composé selon la revendication 1, caractérisé en ce que en ce que K est un reste de formule générale

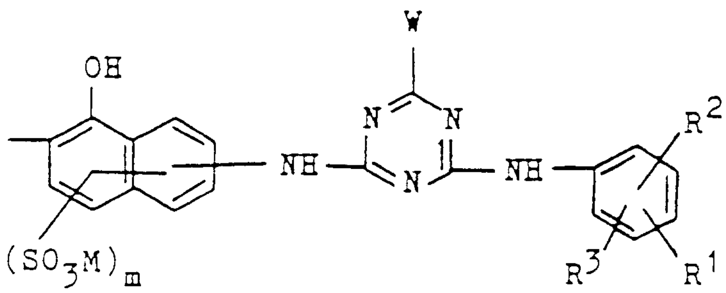

(4v)

dans laquelle m représente le nombre 1 ou 2, M a la signification indiquée dans la revendication 1, W est un groupe sulfo, un groupe alkylsulfonyle de 1 à 4 atomes de carbone, le groupe phénylsulfonyle ou un atome de brome, de fluor ou de chlore, $R^1$ est le groupe $-SO_2-Y$ où Y a l'une des significations données dans la revendication 1, $R^2$ est un atome d'hydrogène, un groupe alkyle de 1 à 4 atomes de carbone, un groupe alcoxy de 1 à 4 atomes de carbone, un atome de chlore ou de brome ou un groupe carboxy, sulfo ou nitro, et $R^3$ représente un atome d'hydrogène.

**5.** Composé selon la revendication 1, caractérisé en ce que K est un reste de formule générale

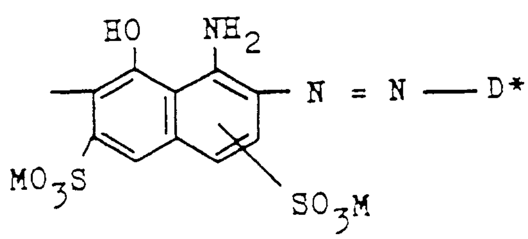

(4p')

dans laquelle M a l'une des significations indiquées dans la revendication 1 et D* est un reste phényle qui peut être substitué par 1, 2 ou 3 substituants du groupe constitué par alkyle de 1 à 4 atomes de carbone, alcoxy de 1 à 4 atomes de carbone, chlore, brome, hydroxy, carboxy, sulfo, carbamoyle, sulfamoyle et alcanoylamino de 2 à 5 atomes de carbone et/ou par un groupe de formule générale $-SO_2-Y$ où Y a l'une des significations indiquées dans la revendication 1, ou est un reste naphtyle qui peut être substitué par 1, 2 ou 3 groupes sulfo et par 1 ou 2 groupes sulfo et 1 ou 2 groupes de formule générale $-SO_2-Y$ où Y a l'une des significations indiquées dans la revendication 1, ou seulement par un tel groupe $-SO_2-Y$.

**6.** Composé selon la revendication 4, caractérisé en ce que W est un atome de fluor ou de chlore.

7. Composé selon la revendication 5, caractérisé en ce que D* est un reste phényle qui est substitué, en position para par rapport au groupe azoïque, par un groupe de formule générale $-SO_2-Y$ où Y a l'une des significations indiquées dans la revendication 1.

8. Composé selon l'une au moins des revendications 1 à 7, caractérisé en ce que n représente le nombre zéro.

9. Composé selon l'une au moins des revendications 1 à 8, caractérisé en ce que R représente un atome d'hydrogène.

10. Composé selon l'une au moins des revendications 1 à 9, caractérisé en ce que Y représente le groupe vinyle ou un groupe β-sulfatoéthyle.

11. Composé selon l'une au moins des revendications 1 à 10, caractérisé en ce que Y est un groupe β-sulfatoéthyle.

12. Composé selon l'une au moins des revendications 1 à 11, caractérisé en ce que M représente un atome d'hydrogène ou un métal alcalin.

13. Procédé de préparation d'un composé ayant la formule générale (1) indiquée et définie dans la revendication 1, caractérisé en ce que l'on copule un composé de diazonium d'un composé amino de formule générale (5)

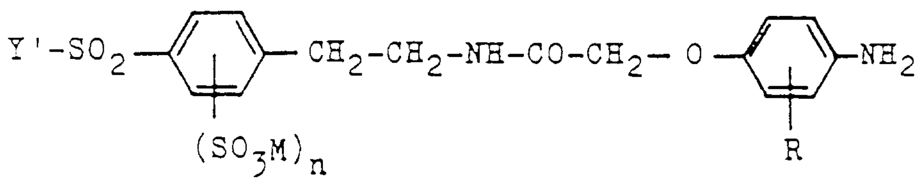 (5)

dans laquelle Y' a l'une des significations de Y ou est le groupe β-hydroxyéthyle et R, M et n ont les significations indiquées dans la revendication 1, avec un constituant de copulation de formule générale H-K où K a la signification indiquée dans la revendication 1, et on fait éventuellement réagir le composé azoïque formé avec un autre composé diazoïque ou constituant de copulation et, au cas où Y' est le groupe β-hydroxyéthyle, on le transforme, dans le composé azoïque formé, en un groupe Y ayant la signification indiquée dans la revendication 1.

14. Utilisation d'un composé correspondant à la formule générale (1) de la revendication 1 pour la teinture (y compris l'impression) d'une matière contenant des groupes hydroxy et/ou carboxamide, en particulier d'une matière fibreuse.

15. Procédé de teinture (y compris d'impression) d'une matière contenant des groupes hydroxy et/ou carboxamide, en particulier d'une matière fibreuse, selon lequel on applique un colorant sur la matière ou on l'y introduit et on le fixe à la chaleur et/ou à l'aide d'un agent à action alcaline, caractérisé en ce que l'on utilise comme colorant un composé correspondant à la formule générale (1) de la revendication 1.

16. Composé correspondant à la formule générale

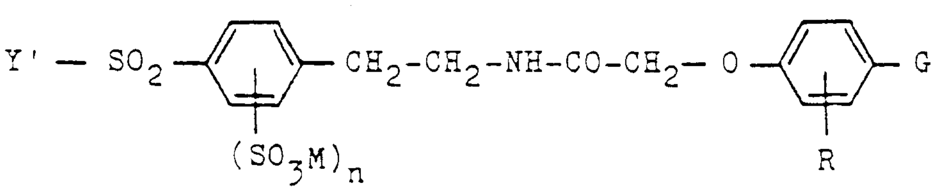 (5')

dans laquelle

- G représente le groupe amino ou le groupe nitro,
- R est un atome d'hydrogène, un groupe nitro, un groupe alkyle de 1 à 4 atomes de carbone, un groupe alcoxy de 1 à 4 atomes de carbone, un groupe carboxy, un groupe hydroxy ou un atome d'halogène,
- M est un atome d'hydrogène ou un atome métallique formant un sel, de préférence un atome de métal alcalin,
- n représente le nombre 0 ou 1 et
- Y' est le groupe β-hydroxyéthyle ou le groupe vinyle ou un groupe éthyle qui contient en position β un substituant qui peut être éliminé par une base avec formation du groupe vinyle.

**17.** Composé selon la revendication 16, caractérisé en ce que Y' représente le groupe β-hydroxyéthyle ou β-sulfatoéthyle ou le groupe vinyle.

**18.** Composé selon la revendication 16 ou 17, caractérisé en ce que n représente le nombre zéro.

**19.** Composé selon l'une au moins des revendications 16 à 18, caractérisé en ce que R est un atome d'hydrogène.

**20.** Procédé de préparation d'un composé selon la revendication 16, caractérisé en ce que l'on fait réagir un composé de formule générale (6)

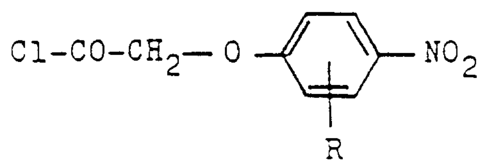

(6)

dans laquelle R a la signification indiquée dans la revendication 1, avec un composé amino de formule générale

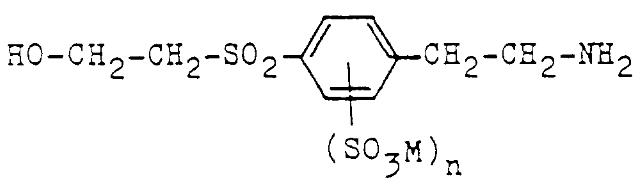

(7)

dans laquelle M et n ont les significations indiquées dans la revendication 1, et on réduit le groupe nitro dans le composé ainis obtenu de formule générale (8)

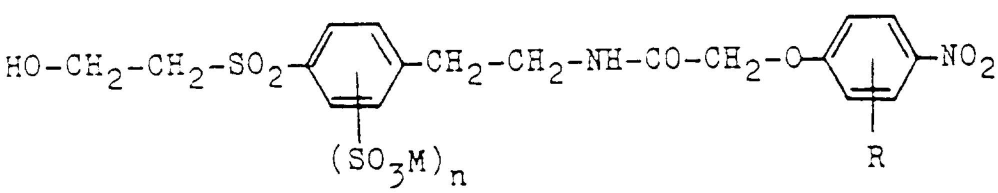

(8)

dans laquelle R, M et n ont les significations indiquées dans la revendication 16, pour former le composé de formule générale (5A)

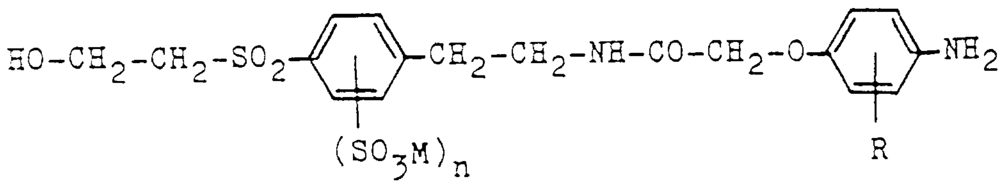

(5A)

dans laquelle R, M et n ont les significations indiquées ci-dessus, et on transforme éventuellement, dans les composés de formule (8) et (5A), le groupe β-hydroxyéthylsulfonyle en un groupe de formule Y-$SO_2$- où Y a l'une des significations indiquées dans la revendication 16.

**21.** Utilisation d'un composé selon la revendication 16 pour la synthèse de colorants, en particulier de colorants azoïques.